(19) Europäisches Patentamt
European Patent Office
Office européen des brevets

(11) **EP 3 384 017 B1**

(12) **EUROPEAN PATENT SPECIFICATION**

(45) Date of publication and mention
of the grant of the patent:
**08.09.2021 Bulletin 2021/36**

(21) Application number: **16822296.6**

(22) Date of filing: **05.12.2016**

(51) Int Cl.:
***C12N 9/00*** *(2006.01)*     ***D01D 5/06*** *(2006.01)*
***D01F 9/00*** *(2006.01)*

(86) International application number:
**PCT/US2016/064939**

(87) International publication number:
**WO 2017/096359 (08.06.2017 Gazette 2017/23)**

(54) **AN ENZYME DELIVERY SYSTEM**

ENZYM BEREITSTELLUNGSSYSTEM

SYSTÈME DE LA LIVRAISON DES ENZYMES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.12.2015 US 201562262619 P**

(43) Date of publication of application:
**10.10.2018 Bulletin 2018/41**

(73) Proprietor: **DuPont US Holding, LLC
Wilmington, DE 19805 (US)**

(72) Inventor: **SAQUING, Carl
Newark, Delaware 19713 (US)**

(74) Representative: **Dehns
St. Bride's House
10 Salisbury Square
London EC4Y 8JD (GB)**

(56) References cited:
**WO-A1-2010/062745     WO-A1-2015/177077
US-A1- 2007 134 305     US-A1- 2007 158 880
US-A1- 2011 226 690     US-B1- 8 641 960**

**Description**

CROSS REFERENCE TO RELATED APPLICATIONS

This application claims benefit of U.S. Patent Application No 62/262619, filed December 3, 2015.

FIELD OF DISCLOSURE

This disclosure relates generally to an enzyme delivery system and more specifically to enzymes encapsulated in fibers.

BACKGROUND OF THE DISCLOSURE

Enzymes are commonly required as catalysts in various industries. However, enzymes have limited application and shelf life due to their instability. Enzyme activity generally decreases during storage or processing making their use in many processes difficult.

Enzymes are often supplied in liquid formulations. Liquid formulations are preferred in many cases for several reasons, including solubility, convenience in handling (e.g., dispensing, pouring, pumping or mixing), and compatibility with existing manufacturing processes, which are typically aqueous processes.

Enzymes are often biochemically less stable in aqueous liquids. When an enzyme is added to an aqueous medium without steps taken to stabilize the enzyme, the enzyme typically is rapidly denatured in the water. Enzymes may hydrolyze in water and often will degrade itself or other enzymes that may be present. In the aqueous state, undesirable reactions (e.g., proteolysis, premature catalytic conversion of substrates, loss of cofactors, oxidation) often occur at unacceptable rates. Aqueous enzyme formulations can also exhibit signs of physical instability, including the formation of precipitates, crystals, gels, or turbidity, during extended storage. Consequently, a loss of enzyme activity is observed over time.

Encapsulation of enzymes in solid particles is known in the art, see for example WO 2015/177077, however such encapsulates are typically of such a size that renders them optically visible and prone to sedimentation or stratification, hence problematic for formulators of liquid products such as detergents, on both aesthetic and functional grounds

Thus, there is a need for enzymes that are not prone to sedimentation or stratification and retain their activity for long periods of time (shelf-storage), particularly for aqueous compositions. It is also desirable for some applications that the encapsulated enzyme is not visible in the end use formulation or composition.

SUMMARY

The present disclosure is directed to an enzyme delivery system comprising:

a plurality of electroblown fibers comprising:

a) a water soluble polymeric resin; and
b) an enzyme;

wherein the enzyme is encapsulated in the electroblown fibers and is present in an amount from 0.1 to 40 wt% based on total weight of the electroblown fibers;
wherein activity of the enzyme after encapsulation is from 50 to 100%; and wherein at least a portion of the electroblown fibers have a diameter of 25 microns or less.

BRIEF DESCRIPTION OF FIGURES

Figure 1 shows a top view of solubility behavior of polyvinyl alcohol (PVA) fiber mats having PVA content ranging from 0.05 wt% to 2.5 wt% based on the total weight of the solution in solutions of water, ethanol, and mixtures of water and ethanol as shown.

Figure 2 shows top and side views of the solubility behavior of PVA fiber mats placed in water, propylene glycol, and mixtures of water and propylene glycol containing no enzyme.

Figure 3 shows top and side views of the solubility behavior of PVA fiber mats in solutions of water, propylene glycol, and mixtures of water and propylene glycol where the PVA fiber mats contained 2.2 wt% encapsulated perhydrolase variant based on the total weight of the fiber mat.

DETAILED DESCRIPTION

Definitions

As used herein, the terms "comprises," "comprising," "includes," "including," "has," "having" or any other variation thereof, are intended to cover a non-exclusive inclusion. For example, a method, process, article, or apparatus that comprises a list of elements is not necessarily limited only to those elements but may include other elements not expressly listed or inherent to such method, process, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components of the invention. This is done merely for convenience and to give a general sense of the invention. This description should be read to include one or at least one and the singular also includes the plural unless it is obvious that it is meant otherwise.

The term "fiber" or "fibers" as used herein is intended to mean any structure (e.g. a matrix or coated structure) that has an aspect ratio (L/D) of at least 5.

The term "web", "fiber mat", "fiber matrices" as used herein is intended to mean a structure comprising more than one fiber, where the fibers are in proximity or contact with one another at one or more points.

The term "solution spun" or "solution spinning" as used herein is intended to mean the formation of fibers by extruding a solution of a polymer composition from a spinneret or spin pack to form fine streams of fluid. The specific method of solution spinning used herein is electroblowing from polymer solution and is described in more detail hereinafter.

The term "encapsulated", "encapsulate," "encapsulates," "encapsulation" or other similar terminology as used herein refers to at least partially or completely surrounding or associating an active substance (e.g. an enzyme) with another material (e.g., polymeric matrix) to prevent or control the release of active, for example, within an aqueous composition.

The term "encapsulation efficiency" as used herein means the percent of enzyme solids (active and inactive) by mass that gets incorporated in the delivery system relative to the total mass of enzyme solids contained in the starting spinning solution.

The term "enzyme payload" or "enzyme activity" as used herein means the concentration in mass of active enzyme that is encapsulated in the delivery system. It can be expressed in activity units, but preferably is expressed gravimetrically in units of mg/g (mg active enzyme per gram of delivery system).

The term "encapsulation yield" is the mass percentage of active enzyme that is recovered from the delivery system after encapsulation. The payload of a delivery system which achieves a theoretical encapsulation yield of 100% is known as the "theoretical payload".

The term "fragmented web" or "fragmented non-woven web" or "fragmented woven web" as used herein is intended to mean a web of fibers broken apart from a larger web either mechanically (for example by milling) or chemically where the fragmentation is effected through a chemical agent (example by partial dissolution) or a combination of mechanical and chemical means. The fragmented web may be in the form of powders or particles or powdery particulates to the naked eye, but when seen using a high resolution microscope (for example scanning electron microscope), the fragmented web appears fibrous in morphology.

The term "suspension" or "suspended" as used herein refers to a two phase system where a discontinuous solid phase (e.g., fibers) is dispersed within a continuous liquid phase.

The term "soluble" or "solubility" for the purpose of the present disclosure is intended to mean completely in solution at the molecular level or partially in solution. "Partially in solution" means the amount or fraction of the material (e.g., polymer) present in a supernatant resulting from centrifugation. Solubility can be measured, for example, by measuring the material (e.g., polymer) that remains in the supernatant after centrifuging an aqueous suspension containing the material (for example, the material can be a plurality of solution spun fibers, such as a fiber mat with or without enzyme).

The term "dissolution" or "dissolve" or similar terminology used herein refers to a process where solution spun fibers or fiber delivery system becomes soluble.

When an amount, concentration, or other value or parameter is given as either a range or a list of upper values and lower values, this is to be understood as specifically disclosing all ranges formed from any pair of any upper range limit or value and any lower range limit or value, regardless of whether ranges are separately disclosed. Where a range of numerical values is recited herein, unless otherwise stated, the range is intended to include the endpoints thereof, and all integers and fractions within the range. Numerical values are to be understood to have the precision of the number of significant figures provided. For example, the number 1 shall be understood to encompass a range from 0.5 to 1.4, whereas the number 1.0 shall be understood to encompass a range from 0.95 to 1.04, including the end points of the stated ranges. It is not intended that the scope of the invention be limited to the specific values recited when defining a range.

In describing certain polymers, it should be understood that sometimes applicants are referring to the polymers by the monomers used to make them or the amounts of the monomers used to make them. While such a description may not include the specific nomenclature used to describe the final polymer or may not contain product-by-process terminology, any such reference to monomers and amounts should be interpreted to mean that the polymer is made from those monomers, unless the context indicates or implies otherwise.

The materials, methods, and examples herein are illustrative only and, except as specifically stated, are not intended to be limiting. Although methods and materials similar or equivalent to those described herein can be used, suitable methods and materials are described herein.

The present disclosure is directed to an enzyme delivery system. The enzyme delivery system comprises a plurality of electroblown fibers. The electroblown fibers comprise a water soluble polymeric resin and an enzyme. The enzyme is encapsulated in the electroblown fibers. The enzyme delivery system of the present disclosure has excellent encapsulation efficiency, low enzyme leakage over time and the enzymes retain activity.

Fibers

The fibers useful in the present disclosure are produced using a water soluble resin or mixtures of water soluble resins. The fibers can be produced using a mixture of at least one water soluble resin and a non-water soluble resin such that amounts of each are tailored to achieve the desired solubility in aqueous compositions (quick release or controlled release).

In one embodiment, water soluble polymer resins useful in the present enzyme delivery systems are those that when in fiber form have limited solubility so as to prevent or inhibit release or leakage of encapsulated enzyme (the desired leakage described in further detail hereinafter) in aqueous compositions containing up to about 70 weight percent water based on the total weight of the aqueous composition. However, when such aqueous compositions are diluted with water to provide water concentrations greater than 90 wt% and approaching 100 wt% water (such as conditions encountered in a washing machine) the water soluble polymer resin in the enzyme delivery system has sufficient solubility in water to provide at least about 80 weight percent release of the total active enzyme encapsulated therein, and in other embodiments from 90 weight percent to 100 weight percent release of the total active enzyme encapsulated in the enzyme delivery system.

In some embodiments, the water soluble resin is a water soluble polymeric resin. In some embodiments, the water soluble polymeric resin is selected from methylcellulose, hydroxypropyl methylcellulose, guar gum, alginate, polyvinyl pyrrolidone, polyethylene oxide, polyvinyl alcohol, pullulan, polycaprolactam, polyaspartic acid, polylactic acid or mixtures thereof. In another embodiment, the water soluble polymeric resin is selected from polyvinyl pyrrolidone, polyvinyl alcohol, pullulan or mixtures thereof.

In yet another embodiment, the water soluble polymeric resin is fully hydrolyzed polyvinyl alcohol. Fully hydrolyzed is intended to mean 98% hydrolyzed or greater. In yet another embodiment, the water soluble polymeric resin is fully hydrolyzed polyvinyl alcohol having a crystallinity from 20 to 54%. In another embodiment, the water soluble polymeric resin is fully hydrolyzed polyvinyl alcohol having a crystallinity between and optionally including any two of the following: 20, 25, 30, 35, 40, 45, 50 and 54%. In yet another embodiment, the water soluble polymeric resin is fully hydrolyzed polyvinyl alcohol having a crystallinity less than 35%. In yet another embodiment, the water soluble polymeric resin is fully hydrolyzed polyvinyl alcohol having a crystallinity less than 30%.

In some embodiments, the water soluble resin is a polysaccharide or any other naturally occurring resin that can be solution spun into fibers.

Some enzymes, as well as other active agents, cannot withstand the high temperatures necessary for melt spinning fibers. The fibers of the present disclosure are solution spun fibers and specifically are electroblown. The terms "electroblown" or "electroblowing" or "electroblown spinning" may be used interchangeably and is intended to mean where a polymer-enzyme solution is fed towards a spinning nozzle, discharging the polymer solution via the spinning nozzle or spinneret, which is charged with a high voltage, while injecting compressed air via the lower end of the spinning nozzle, and collecting fiber spun, typically in the form of a web. Examples of techniques for electroblowing are disclosed in for example US Patent Nos. 7,618,579 and 7,582,247.

In some embodiments, at least a portion of the solution spun fibers have a diameter of 25 microns or less. In another embodiment, at least a portion of the solution spun fibers have a diameter of 20 microns or less. In another embodiment, at least a portion of the solution spun fibers have a diameter of 5 microns or less. In another embodiment, at least a portion of the solution spun fibers have a diameter of 4 microns or less. In another embodiment, at least a portion of the solution spun fibers have a diameter of 1 microns or less.

In some embodiments, the solution spun fibers have an average fiber diameter between and optionally including any two of the following: 0.30, 0.33, 0.40, 0.50, 0.60, 0.70, 0.80, 0.90, 1.00, 1.10, 1.20, 1.30, 1.34, 1.40 and 1.50 microns. In some embodiments, the solution spun fibers have an average fiber diameter from 0.30 to 1.50 microns. In some embodiments, the solution spun fibers have an average fiber diameter from 0.33 to 1.34 microns.

In some embodiments, the solution spun fibers have an average fiber diameter of 1 microns or less. In some embodiments, the solution spun fibers have an average fiber diameter of 0.85 microns or less. In some embodiments, the solution spun fibers have an average fiber diameter of 0.42 microns or less. In some embodiments, the solution spun fibers have an average fiber diameter 0.34 microns or less.

In some embodiments, at least 15% of the solution spun fibers have an average fiber diameter of 0.81 micron or less. In another embodiment, at least 30% of the solution spun fibers have an average fiber diameter of less than 0.81 micron. In another embodiment, at least 34% of the solution spun fibers have an average fiber diameter of less than 0.81 micron. In another embodiment, at least 50% of the solution spun fibers have an average fiber diameter of less than 0.81 micron.

In another embodiment, at least 95% of the solution spun fibers have an average fiber diameter of 0.31 microns or less. In another embodiment, at least 98% of the solution spun fibers have an average fiber diameter of 0.31 microns or less.

The solution spun fibers of the present disclosure in some embodiments are collected in the form of a fiber mat or web.

Fiber solubility

It was surprising that electroblown solution spun fibers having average diameters as disclosed above and made from water soluble polymeric resins were not soluble in an aqueous composition having up to 70% water based on the total weight of the aqueous composition. It was also surprising that the electroblown solution spun fibers of the present disclosure were soluble in aqueous compositions having greater than 70% water and at temperatures from 30 to 0 degrees C.

There is a great need for encapsulated enzymes that are soluble in water at temperatures below 30 degrees C, known as cold water cleaning technology as well as a need for enzymes that are not prone to sedimentation or stratification. For laundry detergent, a reduction in water temperature requirements can have tremendous impact on energy savings associated with heating water. According to estimates by the UK government in 2010, the average UK washing machine is used for 270 wash cycles per year with each cycle using 16 L of water (Engineering and Physical Sciences Research Council, UK). The cost of heating that water (assuming 2010 10£/kWh and 2.4 M households) is estimated at around £184 MM, or in US dollars, 288 MM (at current exchange rate). In addition, according to estimates in 2007, a reduction of wash temperature from 40°C to 30°C can save 1) a household around 30% on electricity per wash; 2) in Denmark about 65 million kWh, corresponding to 15,000 households annual consumption, and 3) in Europe about 4 billion kWh.

One solution to cold water temperature cleaning is the use of liquid detergent formulations. While liquid formulations work well in cold water, and solve earlier issues with powder clumping and inadequate dissolution in cold water, liquid formulations pose their own problems. With liquid formulations that contain significant amounts of water, the product packages become large, heavy and bulky, requiring high transportation costs. Further, liquid detergent packages necessitate large volumes of display space at stores and corresponding storage by consumers after purchase.

Incumbent detergent formulations are a complex mixture of reagents, but in terms of their cleaning composition they generally contain one or more of the following: 1) one or more surfactants to solubilize fabric-based stains; 2) one or more builders, 3) one or more antiredeposition aids, 4) one or more enzymes to digest stains, 5) one or more non-aqueous solvents (such as propylene glycol), 6) water, and 7) one or more bleaches to degrade and increase the hydrophilicity of colored stains. In an embodiment of the present disclosure the enzyme delivery system is useful in the above cleaning compositions where the detergent is a liquid for laundry or dishes. Washing at lower temperatures (below 35 °C) pose problems of removing fats and oils from fabrics or household utensils. Although this can be addressed by using in the liquid formulations enzymes that degrade fats and oils, an element that needs to be addressed is the incompatibility of these enzymes with other enzymes and other components in the formulation. To resolve incompatibility issues, usually enzymes and components are encapsulated in powders or films where the encapsulants are usually water-soluble polymers and the size dimensions are in microns or above. At these dimensions, however, these formulations typically require above ambient temperature water to be effective because of the limited solubility and dissolution kinetics of the polymers at room temperature (20-26 °C) or lower than typical room temperature during the cold season (1-20 °C) in other parts of the world. The present disclosure addresses this problem by providing enzymes that are encapsulated in fiber matrices that are soluble at cold water temperatures (e.g., from about 0 to about 30 degrees C).

Polyvinyl alcohol (PVA) fiber matrices (also referred to herein as a "web" or "fiber mat") with and without enzyme can have different solubility and fragmentation behavior in water and water-organic solvent mixtures including water-propylene glycol and water-ethanol mixtures at different water content. These behaviors are related to fiber size and fiber crystallinity and have an impact on enzyme leakage.

PVA solubility in water has been known to be affected by degree of hydrolysis of PVA (Briscoe B, Luckham P, Zhu S. The effects of hydrogen bonding upon the viscosity of aqueous poly(vinyl alcohol) solutions. Polymer. 2000;41:3851-3860). As the degree of hydrolysis is increased, the amount of the hydrophobic acetate groups are decreased, hence the PVA solubility is increased. In general, PVA, with a degree of hydrolysis below 70 %, becomes insoluble. As you increase above 70 % degree of hydrolysis, PVA solubility increases up to a maximum (ca. 90 %) at which the PVA solubility starts to decrease due to overpowering effect of strong inter and intra chain hydrogen bonding making the polymer highly crystalline. It has been found that for enzymes encapsulated in a PVA fiber mat, leakage of the encapsulated enzyme can be controlled by PVA polymer crystallinity and PVA fiber size.

The % crystallinity of the fiber can be determined using dynamic scanning calorimetry (DSC) according to techniques known to those skilled in the art. The crystallinity of PVA fiber and powder was determined according to the procedure described in Example 6 using a Q1000 Modulated DSC from TA Instruments.

In some embodiments, the solution spun fibers have a crystallinity from 20% to 54%. In some embodiments, the solution spun fibers have a crystallinity from 20% to 54% and the water soluble polymeric resin is a fully hydrolyzed polyvinyl alcohol.

In some embodiments, the solution spun fibers have a crystallinity less than 35%. In some embodiments, the solution spun fibers have a crystallinity of less than 35% and the water soluble polymeric resin is a fully hydrolyzed polyvinyl alcohol.

Examples of suitable PVA that may be used for solution spun fibers useful in the present application include ELVANOL 80-18 and ELVANOL 70-30. ELVANOL 80-18 is a 98 to 98.8 % hydrolysed copolymer of PVA (polyvinyl alcohol) and another monomer and available from Kuraray Co., Ltd. ELVANOL 70-03 is a 98 to 98.8 % hydrolysed PVA (polyvinyl alcohol) and also available from Kuraray Co., Ltd.

As further described in Example 6, the solubility behavior in water of the PVA fiber mats in comparison to PVA powders as obtained from the manufacturer was determined by visual inspection and quantitatively. Both ELVANOL 70-03 and ELVANOL 80-18 as obtained from the manufacturer do not dissolve or disintegrate in water at room and cold temperature.

When transformed into fibers, it was surprisingly discovered that there was rapid fragmentation and eventual visual disappearance of the polyvinyl alcohol fibers in water.

The solubility behavior of the fibers were determined quantitatively according the procedure described in Example 6 where a known amount of fiber was placed in a solution consisting of water and propylene glycol ranging from: 0 wt% water/100 wt% propylene glycol to 100 wt% water/0 wt% propylene glycol at a controlled temperature and fiber concentration. The solution was held for a desired time at a desired temperature and was then centrifuged and the supernatant was analyzed for PVA content using the spectrophotometric method described in the published journal article "Simple spectrophotometric method for determination of polyvinyl alcohol in different types of wastewater, L. Procházková, Y. Rodríguez-Muñoz, J. Procházka, J. Wannera, 2014, Intern. J. Environ. Anal. Chem., 94, 399-410". This method is based on complexation with iodine according to the Pritchard method that has been previously described in earlier publications including the following: I.F. Aleksandrovich and L.N. Lyubimova, Fibre Chem. 24, 156 (1993); D.P. Joshi, Y.L. Lan-Chun-Fung and J.G. Pritchard, Anal. Chim. Acta. 104, 153 (1979); Y. Morishima, K. Fujisawa and S. Nozakura, Polym. J. 10, 281 (1978); J.G. Pritchard and D.A. Akintola, Talanta. 19, 877 (1972).

As described in Example 6, PVA fiber mats placed in water was no longer visible with the naked eye within 2 minutes at room temperature (~25°C), between 2.5 to 5 min at 15°C, between 3 to 7 min at 10°C, between 5 to 10 min at 5°C and between 10 to 20 min at close to 0°C. Not being bound to any particular theory, two possible factors may have contributed to this: the high surface area of the PVA fibers compared to the PVA powders and the decreased crystallinity of the PVA chains in the fibers. The fiber mat solubility properties can advantageously be used in cold water cleaning technology including cold water laundry detergent applications.

In some embodiments, the solution spun fibers have a solubility of 7.7 mg/mL or less in water at a temperature from 30 to 0 degrees C. In some embodiments, solution spun fibers of fully hydrolyzed polyvinyl alcohol have a solubility of 7.7 mg/mL or less in water at a temperature from 30 to 0 degrees C.

Enzyme

The enzyme used will be dependent on the desired use of the enzyme delivery system. In some embodiments, the enzyme delivery system may be used for antimicrobial uses. In another embodiment, the enzyme delivery system may be used in cleaning compositions, such as but not limited to, bleaching or disinfecting compositions for glass, wood or metal, dish detergent, laundry detergent, contact solution, toothpaste and the like.

In some embodiments, the enzyme is selected from peroxidases, gluco-amylases, xylanases, hemicellulases, cellulases, lipolytic enzymes, metallolipolytic enzymes, pectate lyases, metalloproteinase, amadoriase, arabinofuranosidases, phytases, isomerases, lipases, phospholipases, esterases, cutinases, pectinases, keratanases, reductases, oxidases, phenoloxidases, lipoxygenases, ligninases, pullulanases, tannases, pentosanases, malanases, beta-glucanases, arabinosidases, hyaluronidase, chondroitinase, dextranase, transferase, laccase, mannanase, xyloglucanases, or mixtures thereof.

In some embodiments, a mixture of enzymes may be used. In another embodiment, the enzyme is perhydrolase, protease, amylase or mixtures thereof.

In some embodiments, the enzyme is selected from esterases, serine hydrolases, acyl transferases, glycosyltransferases, aryl esterases, pectinases, catalases or mixtures thereof.

In some embodiments, the enzyme is present in an amount from 0.1 to 40 wt% based on total weight of the solution spun fibers. In some embodiments, the enzyme is present in an amount between and including any two of the following: 0.1, 0.5, 1, 2, 3, 4, 5, 10, 15, 20, 25, 30, 35 and 40 wt% based on total weight of the solution spun fibers. In another embodiment, the enzyme is present in an amount from 0.1 to 30 wt% based on total weight of the solution spun fibers. In another embodiment, the enzyme is present in an amount from 0.1 to 15 wt% based on total weight of the solution spun fibers. In some embodiments, the enzyme is present in an amount between and including any two of the following: 0.1, 0.5, 1, 2, 3, 4, 5, 10 and 15 wt% based on total weight of the solution spun fibers.

One skilled in the art could envision encapsulation of other active agents in addition to enzymes, such as, but not limited to proteins, nucleic acid molecules, peptides, yeast or bacteria that would benefit from encapsulation in solution spun fibers, as well as benefit from encapsulation in solution spun fibers made from water soluble polymeric resins.

Detergents compositions often contain enzymes (e.g., a protease) to aid in the degradation and removal of enzyme sensitive stains, soils and deposits. Enzymes for inclusion in liquid detergent compositions are well known and may be any enzyme suitable for use in detergent compositions, such as but not limited to proteases, amylases, cellulases, lipases, xylanases, mannanases, cutinases, pectinase, oxidases or mixtures thereof. Enzymes particularly suitable for detergent applications are proteases such as subtilisin, and amylases such as those from the *Bacillus* species.

Formulations or compositions which contain enzymes experience the problem of decreased enzyme activity over time, especially liquid detergents which contain high levels of surfactant and water. Surfactants, for example alkyl sulfates, tend to deactivate enzymes and render them inactive. As a result, the detergent may have an expiration date and/or increased amounts of costly enzyme need to be added to increase shelf life. Thus, the enzyme delivery system of the present disclosure is well suited for use in liquid cleaning compositions such as liquid detergent compositions.

Encapsulation efficiency is the percent of mass of enzyme solid (active and inactive) that is encapsulated in the enzyme delivery system based on the total mass of enzyme solid (active and inactive) contained in the starting solution for spinning. Encapsulation efficiency may be calculated as:

$$\text{Encapsulation Efficiency (\%)} = 100 \ X \ \frac{\text{(Total amount of enzyme in fiber mat)}}{\text{(Total amount of enzyme in spin solution)}}$$

A higher encapsulation efficiency percentage implies a greater amount of the starting enzyme was encapsulated. For example, 100% efficiency means that all the enzyme in the starting solution was encapsulated in the fibers. As is further described in Example 1 herein, it was found that the encapsulation efficiency may be 95% $\pm$ 10%. It is believed that the difference between 100% efficiency versus what was observed could be within statistical or random error. Thus, the method of enzyme encapsulation described herein may be more efficient than conventional encapsulation methods for enzymes like spray drying and fluidized bed processes.

Enzyme activity and leakage

It is also advantageous that the enzyme activity (enzyme payload) after encapsulation be retained. The term "enzyme payload" or "enzyme activity" means the concentration in mass of active enzyme that is encapsulated in the delivery system. Typically, it is expressed as mass of active enzyme per total mass of the delivery system. The term "% enzyme activity after encapsulation" as recited herein refers to the encapsulation yield of active enzyme. The "% enzyme activity after encapsulation" or "encapsulation yield" is the enzyme activity or enzyme payload after encapsulation divided by the starting enzyme activity or starting enzyme payload before encapsulation expressed as a percentage. The starting enzyme activity or starting enzyme payload before encapsulation is sometimes referred to as the theoretical payload. The enzyme activity of the encapsulated enzymes can be determined using techniques well known to those skilled in the art. The enzyme activity herein was determined by adding the enzyme delivery system (e.g., fiber mat having encapsulated enzyme) to a suitable liquid where all the enzyme is released or is made accessible for example with vigorous stirring (such as by relative centrifugal force of more than 4000) and then is assayed for enzyme. An example of this determination is found in Example 3, and Table 7 where the AAPF-pNA assay was used to measure enzyme activity of protease that had been encapsulated in electroblown fibers of PVA.

In some embodiments, percentage of active enzyme after encapsulation is between and including any two of the following: 50, 60, 65, 70, 75, 80, 85, 90, 95 to 100%.

In some embodiments, the percentage of active enzyme after encapsulation is from 50 to 100% or 60 to 100%. In another embodiment, the percentage of active enzyme after encapsulation is from 65 to 95%. In another embodiment, enzyme activity after encapsulation is from 13 to 20 mg/g based on a predetermined enzyme payload of 20 mg/g. In another embodiment, enzyme activity after encapsulation is between and including any two of the following: 13, 14, 15, 16, 17, 18, 19 and 20 mg/g based on a predetermined enzyme payload of 20mg/g.

As is further described in Example 3 herein, it was found that the percentage of active enzyme after encapsulation was at least 95% for protease encapsulated in electroblown fibers of ELVANOL 80-18. The percentage of active enzyme after encapsulation was at least 65% for ELVANOL 70-03 encapsulated protease. The decrease in activity for the protease encapsulated in ELVANOL 70-03 is possibly due to heating the spinning solution of enzyme and ELVANOL 70-03 to a temperature from 30 to 40 degrees C so that the ELVANOL 70-03 remained in solution.

It is also desirable that the amount of enzyme that leaks out of the fibers over time is minimized during its storage. It is also desirable that enzyme release only occurs with the desired trigger.

Enzyme leakage as used herein means the amount or fraction (which can be expressed as a weight percent) of the active enzyme released from a delivery system. In the disclosure herein, enzyme leakage is usually expressed as a mass percent of active enzyme leaked relative to the original amount by mass of active enzyme encapsulated in the delivery system (e.g., a delivery system of solution spun fibers).

The enzyme leakage of an enzyme delivery system can be determined by taking known amounts of the enzyme delivery system which has a known amount of active enzyme (i.e., the initial enzyme payload) and dosing the enzyme delivery system into an aqueous composition. The aqueous composition can be stored under different conditions such as temperature and then analyzed at given time points for enzyme leakage into the aqueous composition. Leakage into the aqueous composition can be determined by techniques known to those skilled in the art. For example, enzyme leakage can be determined by measuring the enzyme activity of the resulting supernatant that remains after centrifuging at moderate conditions (e.g., relative centrifugal force of about 1000) the aqueous composition containing the enzyme delivery system to separate particles from the bulk aqueous composition. As previously mentioned, the analysis for enzyme leakage can be performed at given points in time and under different storage conditions of the aqueous composition.

Specific examples of measuring enzyme leakage are described in Example 3 for enzyme delivery systems dosed in aqueous compositions containing different amounts of water. The enzyme activity was assayed using for example the ThermoScientific Coomasie Plus™ Bradford Assay Kit (ThermoScientific Product 23236).

For the enzyme delivery system of the present disclosure, enzyme leakage is desired to be stable over time. If leakage occurs, it occurs mostly in the first day then remains stable over the next 200 days. In some embodiments, the percent enzyme leakage at day 14 and day 21 is the same as day 200. In other embodiments, there is little or no additional enzyme leakage after day 14. In some embodiments, the total enzyme leakage is 21% or less after 200 days when the enzyme delivery system is suspended in an aqueous composition comprising 35 wt% water or less based on the total weight of the aqueous composition and wherein the aqueous composition is at a temperature from 20 to 30 degrees C. In another embodiment, the total enzyme leakage is 10% or less after 200 days, when the enzyme delivery system is suspended in an aqueous composition comprising 35 wt% water or less based on total weight of the aqueous composition and wherein the aqueous composition is at a temperature from 20 to 30 degrees C. In yet another embodiment, the total enzyme leakage is 4% or less after 200 days when the enzyme delivery system is suspended in an aqueous composition comprising 35 wt% water or less based on total weight of the aqueous composition and wherein the aqueous composition is at a temperature from 20 to 30 degrees C.

In some embodiments, the total enzyme leakage is 21% or less after 21 days, or in other embodiments after 14 days when the enzyme delivery system is suspended in an aqueous composition comprising 35 wt% water or less based on the total weight of the aqueous composition and wherein the aqueous composition is at a temperature from 20 to 30 degrees C.

In some embodiments, the total enzyme leakage is 4% or less after 21 days or in other embodiments after 14 days, when the enzyme delivery system is suspended in an aqueous composition comprising 35 wt% water or less based on the total weight of the aqueous composition and wherein the aqueous composition is at a temperature from 20 to 30 degrees C.

In some embodiments, the enzyme leakage is between and including two of the following: 10, 11, 12, 13, 14, 15, 20, 25, 30, 31, 32 and 35% (based on the initial enzyme payload) after 30 minutes when the enzyme delivery system is suspended in an aqueous composition comprising 70 wt% water or less based on total weight of the aqueous composition and wherein the aqueous composition is at a temperature from 20 to 30 degrees C.

In some embodiments, the enzyme leakage is less than 32% (based on the initial enzyme payload) after 30 minutes when the enzyme delivery system is suspended in an aqueous composition comprising 70 wt% water or less based on total weight of the aqueous composition and wherein the aqueous composition is from at a temperature from 20 to 30 degrees C.

In some embodiments, the enzyme leakage is less than 13 % (based on the initial enzyme payload), after 30 minutes when the enzyme delivery system is suspended in an aqueous composition comprising 70 wt% water or less based on total weight of the aqueous composition and wherein the aqueous composition is at a temperature from 20 to 30 degrees C.

One skilled in the art when reading the specification will understand that it is desired that enzyme leakage from the solution spun fibers is minimized during its storage in the aqueous composition, and that enzyme release only occurs during the actual usage of the aqueous composition for such purposes as cleaning. For example, in the case of a liquid detergent formulation, it is desired that the enzyme leakage from the solution spun fibers is minimized during its storage in a liquid detergent composition containing less than 70 wt% water, or in alternative embodiments containing from about 35 wt% to about 70 wt% or from about 40 wt% to about 70 wt% water, and that enzyme release only occurs in the washing stage where dilution of the liquid detergent composition with water occurs.

The term "trigger" as used herein means the conditions under which the enzyme is desirably released from the enzyme delivery system. For example, the trigger as used herein can mean diluting the aqueous composition containing the enzyme delivery system above some threshold percentage of water to cause release of the enzyme from the enzyme delivery system. The trigger to release the enzyme from the encapsulated fibers will depend on the amount of water and the temperature of the aqueous composition. In some embodiments, an aqueous composition having greater than 70% water and a temperature from 20 to 30 degrees C is the trigger to release of the enzyme from the fibers. In yet another embodiment, an aqueous composition having 40 to 70% water and a temperature greater than 30 degrees C may be the trigger to release of the enzyme from the fibers.

The enzyme delivery system may be stored in an aqueous composition (e.g., a formulation such as a liquid detergent composition) wherein the amount of water is 70% or less. Aqueous compositions containing the enzyme delivery system may be for example aqueous cleaning compositions previously described herein such as liquid laundry detergents and laundry prespotting compositions.

To trigger the release of enzyme from the fibers, the aqueous composition can be diluted in or with water to increase the amount of water to greater than 70% based on the total weight of the diluted solution, thereby triggering release of the enzyme from the fibers. Preferably, once trigger conditions are achieved, it is desired that at least 90 wt% or at least 95 wt% of the enzyme encapsulated in the enzyme delivery system be released in less than 20 minutes or in alternative embodiments less than 15 minutes or 10 minutes.

As previously mentioned, it is surprising that for the enzyme delivery system of the present disclosure, that at a temperature from 30 to 0 degrees C, the enzyme is released from the fibers, resulting in significant reduction in energy usage. This is particularly advantageous for laundry applications. In some embodiments, the trigger can vary with desired end use application. In other embodiments, it is desired that release of all (100 wt%) of the encapsulated enzyme be triggered upon dilution of the aqueous compositions described herein in less than about 15 minutes at temperatures from about 0 to about 30 degrees C.

In some embodiments, the enzyme is provided in a composition that is substantially free of cells or cell debris.

In some embodiments, the enzyme delivery system includes:

a plurality of electroblown fibers comprising:

a) a water soluble polymeric resin; and
b) an enzyme;

where the enzyme is encapsulated in the electroblown fibers and is present in an amount of from 0.1 to 40 wt%, or 0.1 to 30 wt% or 0.1 to 15 wt% based on total weight of the electroblown fibers; the percent of active enzyme after encapsulation is from 50 to 100%; and at least a portion of the electroblown fibers have a fiber diameter of 25 microns or less. In some embodiments of the enzyme delivery system described herein at least 34% of the electroblown fibers have an average fiber diameter of 0.81 microns or less.

In another embodiment, the enzyme delivery system comprises a plurality of electroblown fibers comprising:

a) a water soluble polymeric resin selected from polyvinyl pyrrolidone, polyvinyl alcohol, pullulan or mixtures thereof; and
b) an enzyme;

where the enzyme is encapsulated in the electroblown fibers and is present in an amount from 0.1 to 40 wt% or 0.1 to 15 wt% based on total weight of the electroblown fibers; the percent of active enzyme after encapsulation is from 50 to 100%; and at least a portion of the electroblown fibers have a fiber diameter of 25 microns or less.

In embodiments of any of the enzyme delivery systems described herein where the water soluble polymeric resin includes or is selected from polyvinyl alcohol, the polyvinyl alcohol may be in some instances a fully hydrolyzed polyvinyl alcohol having a crystallinity of 20-54% or in other instances it may be a fully hydrolyzed polyvinyl alcohol having a crystallinity less than 35%.

In some embodiments, the enzyme delivery system comprises:

a plurality of electroblown fibers comprising:

a) a fully hydrolyzed polyvinyl alcohol; and
b) an enzyme;

where the enzyme is encapsulated in the electroblown fibers and is present in an amount from 0.1 to 40 wt% based on total weight of the electroblown fibers; the percent of active enzyme after encapsulation is from 50 to 100%; at least a portion of the electroblown fibers have a fiber diameter of 25 microns or less; and the enzyme leakage is less than 32% or alternatively less than 13% after 30 minutes when the enzyme delivery system is suspended in an aqueous composition comprising 70 wt% water or less based on total weight of the aqueous composition and where the aqueous composition is at a temperature from 20 to 30 degrees C.

In another embodiment, the enzyme delivery system comprises:

a plurality of electroblown fibers comprising:

a) a water soluble polymeric resin selected from polyvinyl pyrrolidone, polyvinyl alcohol, pullulan or mixtures thereof; and
b) an enzyme selected from perhydrolase, protease, amylase or mixtures thereof;

where the enzyme is encapsulated in the electroblown fibers and is present in an amount between and including any two of the following: 0.1, 0.5, 1, 2, 3, 4, 5, 10, 15, 20, 25, 30, 35 and 40 wt% based on total weight of the electroblown fibers; where the percent of active enzyme after encapsulation is between and including any two of the following: 50, 55, 60, 65, 70, 75, 80, 85, 90, 95 and 100%; where at least a portion of the electroblown fibers have a fiber diameter of 25 microns or less, or 1.5 microns or less, or 0.4 microns or less; and/or where the enzyme is provided in a composition that is substantially free of cells or cell debris.

In some embodiments, the enzyme delivery system comprises:

a plurality of electroblown fibers comprising:

a) a water soluble polymeric resin selected from polyvinyl pyrrolidone, polyvinyl alcohol, pullulan or mixtures thereof; and
b) an enzyme selected from perhydrolase, protease, amylase or mixtures thereof;

where the enzyme is encapsulated in the electroblown fibers and is present in an amount from 0.1 to 40 wt% based on total weight of the electroblown fibers; the percent of active enzyme after encapsulation is from 50 to 100%; and at least a portion of the electroblown fibers have a fiber diameter of 25 microns or less.

In some embodiments, the enzyme delivery system comprises:

a plurality of electroblown fibers comprising:

a) a fully hydrolyzed polyvinyl alcohol having a crystallinity of 20-54%; and
b) an enzyme;

where the enzyme is encapsulated in the electroblown fibers and is present in an amount from 0.1 to 40 wt% based on total weight of the electroblown fibers; the percent of active enzyme after encapsulation is from 50 to 100%; and at least a portion of the electroblown fibers have a fiber diameter of 25 microns or less.

Another embodiment of the present disclosure provides an aqueous composition that comprises any of the enzyme delivery systems previously described herein where the aqueous composition comprises 70 wt% or less of water based on total weight of the aqueous composition. In another embodiment of the present disclosure, an aqueous composition is provided that comprises any of the enzyme delivery systems previously described where the composition includes from about 35 wt% to about 70 wt% or from about 40 wt% to about 65 wt% water based on the total weight of the composition.

In another embodiment, the aqueous composition comprises:

a) 95 wt% or less, or alternatively 70 wt% or less of water based on the total weight of the aqueous composition; and
b) the enzyme delivery system which includes a plurality of electroblown fibers comprising:

I. a water soluble polymeric resin; and
II. an enzyme;

where the enzyme is encapsulated in the electroblown fibers and is present in an amount from 0.1 to 40 wt% based on total weight of the electroblown fibers; the percent of active enzyme after encapsulation is from 50 to 100%; and at least a portion of the electroblown fibers have a fiber diameter of 25 microns or less.

In alternative embodiments of the aforesaid aqueous compositions containing an enzyme delivery system, the water soluble polymeric resin may be selected from polyvinyl pyrrolidone, polyvinyl alcohol, pullulan or mixtures thereof; and/or the enzyme may be selected from perhydrolase, protease, amylase or mixtures thereof.

In some embodiments, the solution spun fibers of the enzyme delivery system are in the form of a woven web, fragmented woven web, a non-woven web, a fragmented non-woven web, individual fibers or combinations thereof before adding to the aqueous composition. In some embodiments, the aqueous composition comprises the enzyme delivery system in the form of a woven web, fragmented woven web, a non-woven web, a fragmented non-woven web, individual fibers or combinations thereof, wherein the aqueous composition comprises 70 wt% or less of water based on total weight of the aqueous composition.

In another embodiment, the enzyme delivery system is used in a liquid detergent composition comprising 70 wt% or less of water based on the total weight of the liquid detergent composition and wherein the enzyme is any enzyme suitable for use in a detergent composition.

In another embodiment, the enzyme delivery system is used in a liquid detergent composition comprising 70 wt% or less of water based on the total weight of the liquid detergent composition and where the enzyme includes or is selected from at least one of a protease, amylase, cellulase, lipase, xylanase, mannanase, cutinase, pectinase,oxidase or mixtures thereof, or alternatively the enzyme includes or is selected from at least one of a protease, amylase or mixtures thereof.

In another embodiment, the enzyme delivery system is used in a laundry prespotting composition comprising 70 wt% or less of water based on total weight of the laundry prespotting composition.

The enzyme delivery system may further comprise a substrate, mediator or cofactor for the enzyme. The term "enzyme mediator" means any compound capable of increasing the enzymatic activity. More than one mediator may be used. The term "cofactor" is meant to include coenzymes. More than one cofactor may be used.

Unless otherwise defined, all technical and scientific terms used herein have the same meaning as commonly understood by one of ordinary skill in the art to which this invention belongs. In case of conflict, the present specification, including definitions, will control.

Although methods and materials similar or equivalent to those described herein can be used in the practice or testing of the present invention, suitable methods and materials are described herein.

EXAMPLES

**Description of Polymer Resins**

ELVANOL 80-18 is a 98 to 98.8 % hydrolysed copolymer of PVA (polyvinyl alcohol) and another monomer and is available from Kuraray Co., Ltd. ELVANOL 70-03 is a 98 to 98.8 % hydrolysed PVA (polyvinyl alcohol) and is also available from Kuraray Co., Ltd.

**Fiber Diameter Measurements**

Fiber diameters of the solution spun fibers were measured as follows. Fiber samples were plasma-coated with ~1-3 nm of Osmium using an OPC-80T Osmium Plasma Coater and subsequently analyzed by SEM with a Hitachi SU3500 and Hitachi SU5000 Variable Pressure (VP) microscopes with a thermionic and Schottky-Field emission guns, respectively, operated under pressure in the range of 60-120 Pa at 5-10 kV (SU3500) or 1-5 kV (SU5000 VP). The average fiber diameter was determined by measuring the diameter from at least 100 fibers in each sample.

**Example 1**

Example 1 demonstrates that enzyme encapsulated in fiber showed enzyme activity almost identical to the free enzyme and that the enzyme distribution in the fiber is highly uniform.

The polyvinyl alcohol polymer used was ELVANOL 80-18. One part of liquid perhydrolase variant concentrate (about 3.5 wt%) was added to nine parts of a 15 wt % aqueous ELVANOL 80-18 PVA solution to produce a polymer-enzyme solution. The perhydrolase enzyme-containing polyvinyl alcohol (PVA) fibers are prepared using electroblowing. The polymer-enzyme solution is extruded through a spinneret which is made up of an orifice or capillary nozzle in which a high voltage is applied. As the solution comes out of the tip of the nozzle, compressed air is blown directly toward the solution to form fibers, and the fibers are collected into a web on a grounded collector. As fibers are formed, the solvent evaporates from the solution. The voltage, the enclosure temperature, process air flow rates are operated or set respectively in the range of 20-110 kV, room temperature to 60 °C and 0-20 standard cubic feet per minute (scfm). This corresponded to a resulting fiber mat with 2.2 wt % encapsulated enzyme.

One part of liquid perhydrolase variant concentrate (about 3.5 wt%) was added to ninety parts of a 15 wt% aqueous ELVANOL 80-18 PVA solution and a fiber mat was produced in the same manner as described above. This corresponded to a resulting fiber mat with 0.2 wt % of encapsulated enzyme.

The fiber diameter of the enzyme encapsulated PVA fibers were analyzed according to the procedure previously described. The average fiber diameter was determined by measuring the diameter from at least 100 fibers in each sample. Results are shown in Table 1.

**Table 1**

|  | PVA fibers (0 wt% enzyme) | PVA fibers (0.2 wt% enzyme) | PVA fibers (2.2 wt% enzyme) |
|---|---|---|---|
| Ave. diameter | 359 ± 214 nm | 507 ± 269 nm | 585 ± 336 nm |

The distribution of the enzyme in the fiber mat was determined by directly measuring the protein content using the sodium dodecyl sulfate polyacrylamide gel electrophoresis (SDS-PAGE) protocol or the Laemmli method (Laemmli, U. K. (1970)) Cleavage of structural proteins during the assembly of the head of bacteriophage T4. Nature 227(5259): 680-685). Ten small sections (~0.4 in x ~0.4 in) were cut from different areas of the fiber mat which contained 2.2 wt% of encapsulated perhydrolase variant. The enzyme content of each of the 10 cut sections was measured by SDS-PAGE and compared to the known amount (theoretical amount) of enzyme used in the starting spinning solution. Table 2 provides the results of the SDS-PAGE analysis showing that the enzyme distribution in the fiber mat is highly uniform with an average of 0.86 ± 0.09 μg compared to the theoretical amount of 0.90 μg.

The encapsulation efficiency was determined and is a measure of the amount of enzyme encapsulated in the fiber mat relative to the amount of enzyme used in the starting solution for spinning as previously described. The average encapsulation efficiency of all the measured cut sections of the fiber mat is quite high close to 100% at 95 ± 10%, where the difference could be within statistical or random error, demonstrating that the method of encapsulation may be more efficient than conventional encapsulation methods like spray drying and fluidized bed processes. Results are shown in Table 2.

**Table 2**

| Sample | Enzyme Mass from SDS-PAGE (μg) | Theroretical Mass (μg) | Encapsulation Efficiency (%) |
|---|---|---|---|
| 1 | 0.89 | 0.90 | 99 |
| 2 | 1.06 | 0.90 | 118 |
| 3 | 0.92 | 0.90 | 102 |
| 4 | 0.87 | 0.90 | 97 |
| 5 | 0.86 | 0.90 | 96 |
| 6 | 0.76 | 0.90 | 84 |
| 7 | 0.81 | 0.90 | 90 |
| 8 | 0.75 | 0.90 | 83 |
| 9 | 0.88 | 0.90 | 98 |
| 10 | 0.78 | 0.90 | 87 |
| Average | 0.86 | 0.90 | 95 |
| Std Dev | ± 0.09 | 0.90 | ± 10 |

The activity of the perhydrolase enzyme encapsulated in PVA fibers of the fiber mat containing 2.2 wt% of perhydrolase variant was determined by peracetic acid production in a buffered aqueous medium upon reaction of the fiber mat with triacetin and hydrogen peroxide. A new fiber mat composed of ELVANOL 80-18 with enzyme content of 2.2 wt % was made as described above. Cut electroblown fiber mats composed of sample size ranges from 25 mm$^2$ to 1 cm$^2$ with corresponding weights ranging from 1 to 80 mg were used. The reaction medium contained 4 mL buffer (100 mM sodium phosphate, pH 7.2) containing 150 - 200 mM triacetin and hydrogen peroxide (30 - 200 mM). A quantity of fiber mat equivalent to 80 $\mu$g/mL of perhydrolase enzyme was added to the reaction medium. The reactions were sampled at 0, 5, 15, 30, 60 and 105 minutes by transfer of 180 uL of the reaction solution to a vial containing 20 uL of 1.0 M phosphoric acid to terminate the enzyme reaction. The acid-quenched solutions were centrifuged (12,000 rpm, 5 min). The supernatant (0.010 - 0.050 mL) was transferred to a screw-capped glass HPLC vial containing 0.300 mL HPLC grade water for the subsequent modified Karst derivatization protocol (Pinkernell, U. Effkemann, S. Karst, U. Simultaneous HPLC determination of Peroxyacetic acid and hydrogen peroxide, 1997, Anal. Chem. 69 (17), 3623-3627). To initiate the first Karst derivatization reaction, 0.100 mL of 20 mM MTS (methyl-p-tolyl-sulfide) in acetonitrile was added using a positive displacement pipet. The vials were capped and the contents were gently mixed before a 10-minute incubation in the dark at ca. 25°C. Subsequently, 0.400 mL acetonitrile and 0.100 mL 49 mM TPP (triphenylphosphine) in acetonitrile were added to each vial. The vials were vortexed to mix the contents and the vials were incubated in the dark for 30 minutes at ca. 25°C. An internal standard solution, 0.100 mL of 2.5 mM DEET (N,N-diethyl-m-toluamide) in acetonitrile was then added using a positive displacement pipet, the vials recapped and the contents were vigorously shaken to mix. The samples were evaluated by HPLC and analyzed for MTSO (methyl-tolyl-sulfoxide).

Two control reactions were also conducted to compare the rate and magnitude of perhydrolysis reaction with that of the fiber encapsulated perhydrolase. One control is the perhydrolysis reaction involving a PVA fiber mat of ELVANOL 80-18 without an encapsulated perhydrolase and the other control is the perhydrolysis reaction involving a free enzyme in solution. Table 3 summarizes the results of determining the activity of the enzyme perhydrolase in ELVANOL 80-18 with an enzyme content of 2.2 wt % by measuring the peracetic acid generation. Each measurement is usually an average of at least three replicates. The encapsulated enzyme in fiber showed enzyme activity almost identical to the free enzyme in solution as indicated by the generated peracetic acid in the perhydrolysis reaction which demonstrates that the encapsulation of the enzyme in polymeric fiber by electroblowing process does not have any deleterious effect on the enzyme and that the encapsulated enzyme is accessible when placed in aqueous solution. The fiber containing no enzyme showed quite limited peracetic acid production. Results are shown in Table 3.

**Table 3**

| Time (min) | Peracetic Acid Generation (ppm) | | |
|---|---|---|---|
| | Free enzyme in Solution (control) | Enzyme encapsulated in Fiber | No Enzyme in Fiber (control) |
| 0 | 0 | 0 | 0 |
| 5 | 6564 | 6160 | 135 |
| 15 | 6654 | 6986 | 292 |
| 30 | 6821 | 6347 | 439 |
| 60 | 6346 | 6365 | 494 |

**Example 2**

Example 2 demonstrates 0.17% and 1.7% perhydrolase encapsulated in pullulan fibers where enzyme activity is maintained. Perhydrolase enzyme-containing pullulan fibers were prepared using an electroblowing method as in Example 1. Pullulan is a water-soluble polysaccharide consisting of consecutive maltotriose units bound by $\alpha$-1,6 glucosidic linkages. White powder and odorless food grade pullulan purchased from Hayashibara Co., Ltd. of Japan was dissolved in water with stirring to prepare the pullulan solution. One part of liquid perhydrolase enzyme concentrate (about 3.5 wt%) was added to seven and a half parts of a 20 wt % aqueous pullulan solution. This corresponded to a resulting fiber mat with 1.7 wt % of encapsulated enzyme. In another, one part of liquid perhydrolase enzyme concentrate (about 3.5 wt%) was added to seventy five parts of a 15 wt% aqueous pullulan solution. This corresponded to a resulting fiber mat with 0.17 wt % of encapsulated enzyme. Fiber diameter of the pullulan fiber encapsulated enzymes were analyzed according to the method previously described. The average fiber diameter was determined by measuring the diameter from at least 100 fibers in each sample. Results are shown in Table 4.

**Table 4**

| Pullulan fibers (0 wt% enzyme) | Pullulan fibers (0.17 wt% enzyme) | Pullulan fibers (1.7 wt% enzyme) |
|---|---|---|
| ave diameter 944 ± 540 nm | ave diameter 833 ± 251 nm | ave diameter 417 ± 237 nm |

The activity of the perhydrolase enzyme in pullulan fibers was evaluated using the same procedure as in Example 1. Table 5 summarizes the results of determining the activity of the enzyme perhydrolase encapsulated in the pullulan fibers by measuring the peracetic acid generation. Sample A represents a 6.3 mg pullulan fiber mat cut containing 0.17 wt% of perhydrolase which is equivalent to a total of 0.011 mg of perhydrolase in the perhydrolysis reaction. Sample B represents a 2.7 mg of pullulan fiber mat cut containing 1.7 wt% of perhydrolase which is equivalent to a total of 0.046 mg of perhyrdolase in the perhydrolysis reaction. The data showed that the peracetic acid generation of both samples of pullulan-containing fibers was substantially much higher than the fiber containing no enzyme demonstrating the maintained enzyme activity. The peracetic acid generation increased with increase in enzyme content in the reaction solution. Results are shown in Table 5.

**Table 5**

| Time (min) | Peracetic Acid Generation (ppm) | | |
|---|---|---|---|
| | No Enzyme in Fiber (control) | Sample A Enzyme Encapsulated in Pullulan Fiber (0.17 wt%) | Sample B Enzyme Encapsulated in Pullulan Fiber (1.7 wt%) |
| 0 | 0 ± 0 | 0 ± 0 | 0 ± 0 |
| 1 | 10 ± 3 | 74 ± 8 | 550 ± 25 |
| 5 | 23 ± 1 | 769 ± 209 | 1520 ± 13 |
| 15 | 39 ± 2 | 1070 ± 414 | 1971 ± 71 |
| 31 | 60 ± 2 | 1066 ± 329 | 1920 ± 13 |
| 60 | 103 ± 2 | 1185 ± 510 | 1924 ± 11 |

**Example 3**

Example 3 demonstrates encapsulation of protease in polyvinyl alcohol where enzyme activity is maintained with minimal enzyme leakage where the enzyme leakage after 200 days is about the same as after 1 day.

A. Preparation of Protease Enzyme Containing PVA Fibers

Protease enzyme-containing PVA fibers were prepared using an electroblowing method as in Example 1. One part of liquid protease enzyme concentrate (about 12 wt%) was added to 35.6 parts of a 15 wt % aqueous ELVANOL 80-18 solution. This corresponded to a resulting fiber mat with 2.0 wt % of encapsulated protease.

In another, one part of liquid protease enzyme concentrate (about 12 wt%) was added to 19.6 parts of a 30 wt% aqueous ELVANOL 70-03 solution. This corresponded to a resulting fiber mat with 2.0 wt % of encapsulated protease enzyme.

In another, one part of solid protease enzyme concentrate (about 18 wt%) was added to 22 parts of 15 wt % aqueous ELVANOL 80-18 solution and a fiber mat was produced in the same manner as described above. This corresponded to a resulting fiber mat with 5.1 wt % encapsulated enzyme.

In another, one part of solid protease enzyme concentrate (about 22 wt%) was added to thirteen parts of a 15 wt% aqueous ELVANOL 80-18 solution. This corresponded to a resulting fiber mat with 10.1 wt % of encapsulated protease.

The fiber diameter of the PVA fiber encapsulated enzymes were analyzed according to the procedures previously described herein. The average fiber diameter was determined by measuring at least 150 fibers.

Results are shown in Table 6.

Table 6

| Name | Magnification | Classification | Number | Mean | Median | Std Dev | Min | Max | Sample Composition Represented By | | | |
|---|---|---|---|---|---|---|---|---|---|---|---|---|
| | | | | | | | | | Number | Volume | Surface Area | Specific Surface Area |
| [] | [] | [] | [#] | [μm] | [μm] | [μm] | [μm] | [μm] | [%] | [%] | [%] | [%] |
| PVA 70-03 | 5000x | All Fibers | 177 | 0.278 | 0.226 | 0.199 | 0.061 | 1.485 | | | | |
| | | Nano Fibers <1 μm | 173 | 0.258 | 0.223 | 0.145 | 0.061 | 0.963 | 97.74 | 72.96 | 90.52 | 99.60 |
| | | Micro Fibers 1 - 3 μm | 4 | 1.168 | 1.090 | 0.214 | 1.009 | 1.485 | 2.26 | 27.04 | 9.48 | 0.40 |
| | | Coarse Fibers >3 μm | 0 | | | | | | 0.00 | 0.00 | 0.00 | 0.00 |
| PVA 70-03 2.0% Protease | 5000x | All Fibers | 176 | 0.333 | 0.266 | 0.303 | 0.119 | 3.680 | | | | |
| | | Nano Fibers <1 μm | 174 | 0.310 | 0.265 | 0.156 | 0.119 | 0.983 | 98.86 | 58.72 | 91.89 | 99.82 |
| | | Micro Fibers 1 - 3 μm | 1 | 1.078 | 1.078 | | 1.078 | 1.078 | 0.57 | 3.26 | 1.84 | 0.14 |
| | | Coarse Fibers >3 μm | 1 | 3.680 | 3.680 | | 3.680 | 3.680 | 0.57 | 38.02 | 6.27 | 0.04 |
| PVA 80-18 | 1000x | All Fibers | 350 | 0.901 | 0.788 | 0.407 | 0.213 | 3.005 | | | | |
| | | Nano Fibers <1 μm | 262 | 0.716 | 0.721 | 0.155 | 0.213 | 0.994 | 74.86 | 41.11 | 59.49 | 85.66 |
| | | Micro Fibers 1 - 3 μm | 87 | 1.434 | 1.290 | 0.397 | 1.011 | 2.697 | 24.86 | 56.25 | 39.56 | 14.27 |
| | | Coarse Fibers >3 μm | 1 | 3.005 | 3.005 | | 3.005 | 3.005 | 0.29 | 2.64 | 0.95 | 0.07 |

EP 3 384 017 B1

(continued)

| Name | Magnification | Classification | Number | Mean | Median | Std Dev | Min | Max | Sample Composition Represented By | | | |
|---|---|---|---|---|---|---|---|---|---|---|---|---|
| | | | | | | | | | Number | Volume | Surface Area | Specific Surface Area |
| [] | [] | [] | [#] | [μm] | [μm] | [μm] | [μm] | [μm] | [%] | [%] | [%] | [%] |
| **PVA 80-18 2.0% Protease** | 1000x | All Fibers | 252 | 1.344 | 1.138 | 0.919 | 0.361 | 10.396 | | | | |
| | | Nano Fibers <1 μm | 85 | 0.809 | 0.864 | 0.155 | 0.361 | 0.989 | 33.73 | 8.63 | 20.29 | 47.89 |
| | | Micro Fibers 1 - 3 μm | 158 | 1.425 | 1.290 | 0.406 | 1.002 | 2.954 | 62.70 | 51.93 | 66.44 | 51.23 |
| | | Coarse Fibers >3 μm | 9 | 4.994 | 4.418 | 2.203 | 3.015 | 10.396 | 3.57 | 39.44 | 13.27 | 0.88 |
| PVA 80-18 5.1% Protease | 1000x | All Fibers | 311 | 0.988 | 0.789 | 0.616 | 0.230 | 4.523 | | | | |
| | | Nano Fibers <1 μm | 214 | 0.682 | 0.692 | 0.158 | 0.230 | 0.987 | 68.81 | 24.89 | 47.51 | 83.53 |
| | | Micro Fibers 1 - 3 μm | 91 | 1.530 | 1.356 | 0.483 | 1.020 | 2.862 | 29.26 | 55.53 | 45.31 | 16.06 |
| | | Coarse Fibers >3 μm | 6 | 3.677 | 3.506 | 0.513 | 3.192 | 4.523 | 1.93 | 19.57 | 7.18 | 0.41 |
| PVA 80-18 10.1% Protease | 1000x | All Fibers | 232 | 1.092 | 0.936 | 0.569 | 0.218 | 3.979 | | | | |
| | | Nano Fibers <1 μm | 135 | 0.790 | 0.814 | 0.143 | 0.218 | 0.999 | 58.19 | 24.78 | 42.12 | 71.16 |
| | | Micro Fibers 1 - 3 μm | 91 | 1.376 | 1.183 | 0.415 | 1.012 | 2.642 | 39.22 | 53.49 | 49.46 | 28.16 |
| | | Coarse Fibers >3 μm | 6 | 3.551 | 3.528 | 0.377 | 3.090 | 3.979 | 2.59 | 21.73 | 8.41 | 0.68 |

B. Assessing Protease Leakage in Detergent

Protease leakage from the PVA fibers in a detergent formulation containing 35 wt% water was determined as a function of time (days). Two PVA fiber matrices made each from ELVANOL 80-18 and ELVANOL 70-03 by electroblowing and containing 2.0 wt% and 2.0 wt% encapsulated protease, respectively (as measured by AAPF-pNA assay described below), were cut into small ~2-5 mm square pieces. Known amounts of these cut pieces were added to known amounts of high water detergent, TIDE HDL available from Procter & Gamble (with 35 wt% water content) so that "Sample Dose on Detergent" as shown in Table 8 is defined as sample mass of fiber mat in g divided by the sum of the mass of detergent and mass of sample multiplied by 100 to give a percentage. The enzyme leakage is determined by taking aliquots from the mixture at several time points. These aliquots are centrifuged at moderate conditions of a relative centrifugal force (RCF) of about 1000 to separate particles from the bulk detergent and assayed using N-succinyl-L-alanyl-L-alanyl-L-prolyl-L-phenyl-p-nitroanilide (AAPF-pNA) assay to determine protease activity expressed as mg of protease enzyme per g solution. The initial enzyme payload in mg per g of fiber was determined by assaying the protease activity in the fiber matrix using AAPF-pNA assay as described below. A measure on the effect of the encapsulation process on the stability of the enzyme during encapsulation can also be derived from the determined enzyme payload in the fiber. Since the amount of the enzyme in the fiber is predetermined, the percentage of encapsulated enzyme with maintained activity, that is stable, can be calculated. During storage in between time points, samples were well suspended and not mixed constantly. The enzyme leakage of the encapsulated proteases in PVA fibers was compared to encapsulated particulate subtilisin protease (made from fluid bed technology) and to free protease enzyme for benchmarking.

*N-succinyl-L-alanyl-L-alanyl-L-prolyl-L-phenyl-p-nitroanilide (AAPF-pNA) assay to determine protease activity*

The following reagent solutions were used in the AAPF-pNA assay:
AAPF substrate stock:160 mM (i.e., 100 mg/mL) suc-AAPF-pNA dissolved in dimethylsulfoxide (DMSO), Stability buffer: 100 mM MES (pH 5.5) with 0.005% v/v Tween 80 (may optionally include 10 mM $CaCl_2$), Activity buffer: 100 mM Tris (pH 8.5 or 8.6) with 0.005% v/v Tween-80 (may optionally include 10 mM $CaCl_2$), Assay solution (substrate stock diluted 1:100 into activity buffer): 1.6 mM AAPF-pNA in 100 m1M Tris (pH 8.5 or 8.6).

Procedure: An enzyme standard curve was prepared by making serial dilutions of purified subtilisin protease (0.5-10 ppm) in stability buffer. Test samples were prepared to achieve protease concentrations between 1-10 ppm in stability buffer. Assay solution was prepared by diluting the substrate stock 1:100 with activity buffer. 200 $\mu$L of assay solution was added to each well of a 96-well plate.

The assay was performed by adding 10 $\mu$l of diluted protease enzyme solution to each well of the assay solution plate. The solutions were mixed for 10 seconds, and the absorbance change was measured at 410 nm in a microplate reader at 25°C (set in kinetic mode, over 2 minutes). The subtilisin protease activity (AU = activity units) was calculated as $mOD_{415}$/min x dilution factor, where $mOD_{410}$ refers to the optical density of the reaction product multiplied times 1000 as measured at 410 nm.

The enzyme activity (enzyme payload) of the encapsulated protease in fibers for both PVA ELVANOL 80-18 and 70-03 containing 2 wt % of protease were determined using the AAPF-pNA assay. Vigorous stirring (relative centrifugal force (RCF) of more than 4000) was applied after dosing the protease encapsulated enzyme in fiber to ensure that all the enzyme encapsulated was released or are accessible. The assayed enzyme payload after encapsulation as compared to the predetermined enzyme payload before encapsulation was determined (i.e., the encapsulation yield). The encapsulation yield (percentage of active enzyme after encapsulation) is from 65 % to 95 % as shown in Table 7. The decreased stability for the protease encapsulated in ELVANOL 70-03 is due to heating the spinning solution containing the enzyme at a temperature between 30-40 °C to keep the polymer in solution and reduce polymer precipitation. Heating was not required for the spinning solution involving ELVANOL 80-18.

Results are shown in Table 7.

**Table 7**

| Sample | Theoretical Payload (mg/g) | Assayed Payload (mg/g) | Encapsulation yield (percentage of active enzyme encapsulated) (%) |
|---|---|---|---|
| ELVANOL 80-18 encapsulated protease | 20.0 | 19.0 | 95.0 |

(continued)

| Sample | Theoretical Payload (mg/g) | Assayed Payload (mg/g) | Encapsulation yield (percentage of active enzyme encapsulated) (%) |
|---|---|---|---|
| ELVANOL 70-03 encapsulated protease | 20.0 | 13.4 | 65.0 |

The results of the enzyme leakage test are shown in Tables 8 and 9. As shown in Tables 8 and 9, protease enzyme leakage in electroblown fiber matrices of PVA was significantly reduced. It is for the first time that we demonstrate that enzyme leakage, even after 200 days of storage, was limited to either 4 % in ELVANOL 80-18 fibers and 21 % ELVANOL 70-03 fibers compared to the encapsulated particulate subtilisin protease sample produced from commercial fluid bed technology where enzyme leakage is virtually 100 % as early as 1 day storage.

Table 8

| Sample | Mass Sample (g) | Mass Detergent (g) | Enzyme Payload (mg/g) | Sample Dose on Detergent (%) | Total Enzyme Activity (mg/g) | Measured Protease Activity from Sampled Aliquots after # of days (mg/g) | | | | |
|---|---|---|---|---|---|---|---|---|---|---|
| | | | | | | 0 | 1 | 14 | 21 | 200 |
| 80-18 fiber encaps. protease | 0.65 | 20.1 | 19.0 | 3.0 | 0.60 | 0.002 | 0.027 | 0.024 | 0.026 | 0.025 |
| 70-03 fiber encaps. protease | 0.88 | 20.0 | 13.4 | 4.0 | 0.56 | 0.001 | 0.079 | 0.119 | 0.121 | 0.121 |
| Encaps. particulate subtilisin protease | 0.0956 | 5.0 | 80.0 | 2.0 | 1.50 | 0.004 | 1.530 | 1.570 | 1.500 | - |
| Free subtilisin protease | 0.0867 | 5.0 | 120.0 | 2.0 | 2.05 | 2.000 | 1.990 | 2.010 | 1.950 | - |

**Table 9**

| Sample | Temperature (°C) | pH | Enzyme Leakage after # of Days (%) | | | | |
|---|---|---|---|---|---|---|---|
| | | | 0 | 1 | 14 | 21 | 200 |
| ELVANOL 80-18 fiber encapsulated protease | 20 | 8.5 | 0 | 5 | 4 | 4 | 4 |
| ELVANOL 70-03 fiber encapsulated protease | 20 | 8.5 | 0 | 14 | 21 | 21 | 21 |
| Encapsulated particulate subtilisin protease | 20 | 8.5 | 0 | 102 | 105 | 100 | - |
| Free subtilisin protease | 20 | 8.5 | 98 | 97 | 98 | 95 | - |

C. Determination of Enzyme Leakage in Water-Propylene Glycol Mixtures

Protease leakage from the PVA fibers in a water-propylene glycol mixtures with varying water content was determined. The mixtures ranged from containing 0 wt% water/100 wt% propylene glycol to 100 wt% water/0 wt% propylene glycol as shown in Table 10 and were intended to simulate a simple liquid detergent formulation. PVA fiber matrices made from ELVANOL 80-18 by electroblowing and containing 2.0 wt% and 10.1 wt% encapsulated protease as described above, were cut into small ~2-5 mm square pieces. Known amounts of these cut pieces were dosed into known amounts of water-propylene glycol mixtures at a ratio of 1 part fiber matrix in 28 parts of liquid water-propylene mixture. The mixtures after dosing the encapsulated enzyme in fibers are swirled gently by hand for less than a minute and left to stand without stirring to simulate storage conditions.

The enzyme leakage is determined by taking aliquots from the mixture after a thirty minute time point. These aliquots are centrifuged at moderate conditions of a relative centrifugal force (RCF) of about 1000 to separate particles from the bulk detergent and assayed using the ThermoScientific Coomasie Plus™ Bradford Assay Kit (ThermoScientific Product 23236). Results are presented in Table 10 and they show that at an enzyme payload of 20 mg/g and 100 mg/g, enzyme leakage is minimal. For the fiber with 20 mg/g enzyme payload, enzyme leakage is 12 % or less in mixtures with water content of up to 70 % and only 25 % enzyme leakage in mixtures with water content of 100 %. For the fiber with 100 mg/g enzyme payload, the enzyme leakage is less than 10 % in mixtures with water content up to 40 % and only 30 % enzyme leakage in mixtures with water content ranging from 50 to 100 %. This is an interesting finding since the majority of the encapsulated enzyme are kept within the fiber matrix even at 100 % water content as long as no vigorous stirring is applied. Although not exemplified herein, Applicants believe that the solution spun fibers could be altered in solubility properties by adjusting parameters such as the fiber diameter and/or polymer crystallinity to achieve trigger conditions for releasing enzyme at concentrations above 70 wt% water.

**Table 10**

| Sample | Enzyme payload (mg/g) | Enzyme Leakage after 30 min at different water concentration (%) | | | | | | |
|---|---|---|---|---|---|---|---|---|
| | | 0 $H_2O$ | 15 | 30 | 40 | 50 | 70 | 100 $H_2O$ |
| ELVANOL 80-18 encapsulated protease | 20.0 | 8.0 | 2.7 | 3.4 | 6.1 | 6.4 | 12.1 | 25.5 |
| ELVANOL 80-18 encapsulated protease | 100.0 | 2.5 | 2.5 | 3.1 | 6.1 | 24.4 | 31.6 | 27 |

**Example 4**

Example 4 demonstrates encapsulation of amylase in polyvinyl alcohol.

Amylase enzyme-containing PVA fibers were prepared using an electroblowing method as in Example 1. One part of liquid amylase enzyme concentrate (about 8 wt%) was added to fifteen parts of a 15 wt % aqueous ELVANOL 80-18 solution. This corresponded to a resulting fiber mat with 3.6 wt % of encapsulated amylase enzyme. The surface morphology and fiber diameter of the PVA fiber encapsulated enzymes were analyzed using scanning electron microscopy (SEM) according to the methods previously described herein. The average fiber diameter and standard deviation ($\pm$) were determined by measuring the diameter from at least 474 fibers. Results are shown in Table 11.

Table 11

| Name | Magnification | Classification | Number | Mean | Median | Std Dev | Min | Max | Number | Volume | Surface Area | Specific Surface Area |
|---|---|---|---|---|---|---|---|---|---|---|---|---|
| [] | [] | [] | [#] | [µm] | [µm] | [µm] | [µm] | [µm] | [%] | [%] | [%] | [%] |
| PVA 80-18 3.6% Amylase | 1000x | All Fibers | 474 | 0.746 | 0.702 | 0.285 | 0.229 | 3.691 | | | | |
| | | Nano Fibers <1 µm | 429 | 0.686 | 0.682 | 0.158 | 0.229 | 1.000 | 90.51 | 70.36 | 83.23 | 94.75 |
| | | Micro Fibers 1 - 3 µm | 44 | 1.263 | 1.158 | 0.363 | 1.004 | 2.912 | 9.28 | 25.13 | 15.73 | 5.21 |
| | | Coarse Fibers >3 µm | 1 | 3.691 | 3.691 | | 3.691 | 3.691 | 0.21 | 4.51 | 1.04 | 0.04 |

### Example 5

Example 5 demonstrates solubility behavior of PVA fiber mats with and without enzyme

Solubility Behavior in Water-Alcohol Mixtures

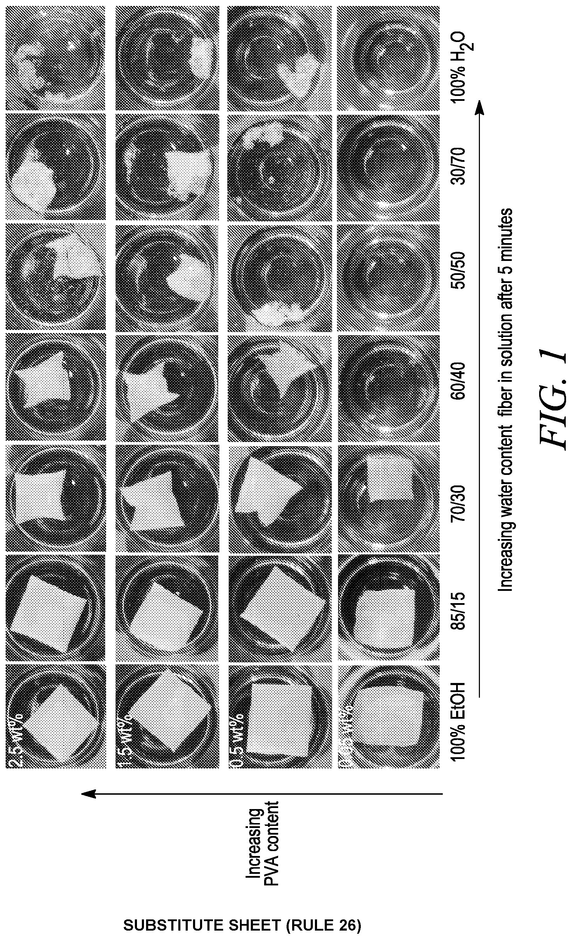

The solubility behavior of the PVA fiber mats placed in water, ethanol, and mixtures of water and ethanol are shown in Figure 1 from a top view perspective. The weight percent of PVA content was varied from 0.05 wt% (bottom row) to 0.5 wt%(second row from bottom) to 1.5 wt% (third row from bottom) to 2.5 wt% (top row). The composition of the solutions tested varied from left to right: 100 wt% ethanol, 85 wt% ethanol/15 wt% water, 70 wt% ethanol/30 wt% water, 60 wt% ethanol/40 wt% water, 50 wt% ethanol/50 wt% water, 30 wt% ethanol/70 wt% water, and 100% water. Figure 1 shows that at a PVA content of 0.05 wt% the PVA fiber mat visually disappears after 5 minutes in solution at water contents of 40 wt% or greater. In the third row from the bottom and top row of Figure 1, a PVA fiber mat of 1.5 wt% and 2.5 wt% PVA content is almost disintegrated at 100 wt% water after 5 minutes in solution, but appears to be largely intact at concentrations up to 70 wt% water. This suggests that enzyme release can be triggered by such variables as the amount of PVA content and weight percent of water in the aqueous solution.

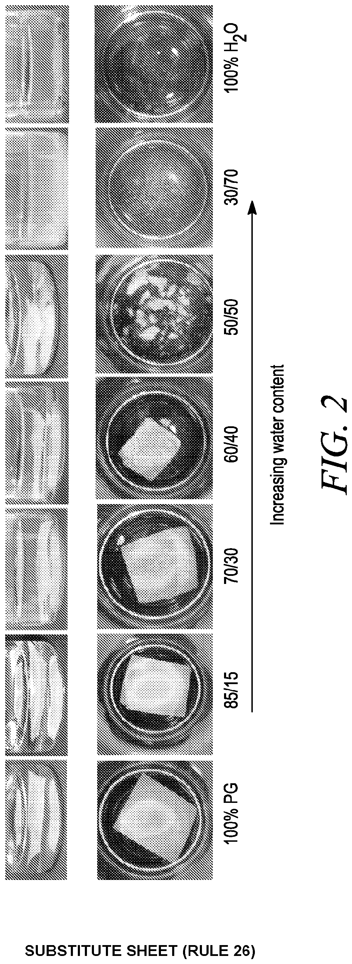

The solubility behavior of the PVA fiber mats placed in water, propylene glycol, and mixtures of water and propylene glycol are shown in Figure 2 from top view and side view perspectives. The composition of the solutions tested varied from left to right: 100 wt% propylene glycol, 85 wt% propylene glycol/15 wt% water, 70 wt% propylene glycol/30 wt% water, 60 wt% propylene glycol/40 wt% water, 50 wt% propylene glycol/50 wt% water, 30 wt% propylene glycol/70 wt% water, and 100% water. Figure 2 shows the PVA fiber mat beginning to disintegrate at 50% water and appears to be in solution at 100 wt% water.

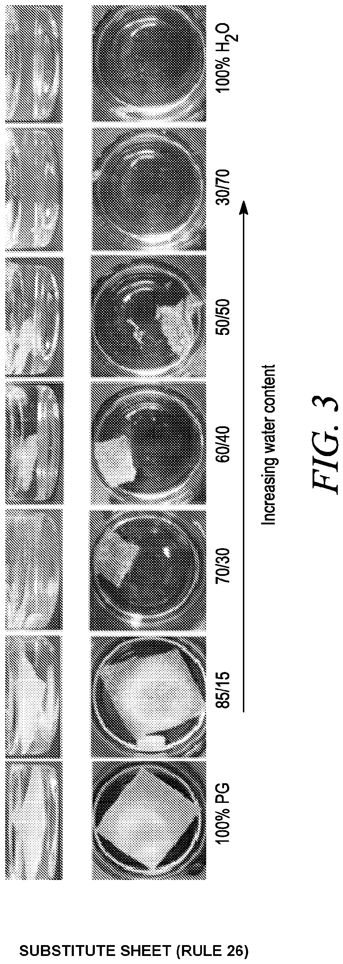

The solubility behavior of PVA fiber mats having encapsulated therein 2.2 wt% perhydrolase variant that were placed in water, propylene glycol, and mixtures of water and propylene glycol is shown in Figure 3 from a top view and side view perspective. The composition of the solutions tested varied from left to right: 100 wt% propylene glycol, 85 wt% propylene glycol/15 wt% water, 70 wt% propylene glycol/30 wt% water, 60 wt% propylene glycol/40 wt% water, 50 wt% propylene glycol/50 wt% water, 30 wt% propylene glycol/70 wt% water, and 100% water. Figure 3 shows the PVA fiber mat beginning to significantly disintegrate at 50% water and appears to be in solution at 100 wt% water.

The PVA fiber mats shown in Figures 1 to 3 are electroblown fiber mat samples made from ELVANOL 80-18, with or without enzyme as indicated above and were prepared as described in Example 1.

The ELVANOL 80-18 PVA fiber mats with or without enzymes do not disintegrate and disappear in propylene glycol and ethanol, and also in mixtures of water and propylene glycol or ethanol having up to 40% water content. Moreover, the disappearance of the fiber mat in 100 % water is a function of the fiber mass to solvent mass ratio or the wt % fiber solids content in the mixture. It can be seen in Figure 1 that above 0.5 wt % PVA, not all the fiber solids disappeared in the solvent mixtures as the water content was increased from 0 to 100 %.

Additionally, it is noted from these figures that it is possible to vary variables such as PVA content, and water content in the formulation to suppress enzyme leakage during storage but to trigger release of the enzyme upon significant dilution with water where the composition of the solution contains almost 100 wt% water.

### Example 6

Example 6 demonstrates a quantitative measurement of PVA solubility that was performed using the spectrophotometric method described in the published journal article "Simple spectrophotometric method for determination of polyvinyl alcohol in different types of wastewater, L. Procházková, Y. Rodríguez-Muñoz, J. Procházka, J. Wannera, 2014, Intern. J. Environ. Anal. Chem., 94, 399-410" based on complexation with iodine according to the Pritchard method that has been previously described in earlier publications including the following: I.F. Aleksandrovich and L.N. Lyubimova, Fibre Chem. 24, 156 (1993); D.P. Joshi, Y.L. Lan-Chun-Fung and J.G. Pritchard, Anal. Chim. Acta. 104, 153 (1979); Y. Morishima, K. Fujisawa and S. Nozakura, Polym. J. 10, 281 (1978); J.G. Pritchard and D.A. Akintola, Talanta. 19, 877 (1972).

Quantitative PVA Solubility Analysis

Electroblown fiber mat samples from ELVANOL 80-18 were prepared as described in Example 1. These fiber mats were vacuum dried overnight, were weighed, and placed in 20-ml scintillation vials. Propylene glycol was purchased from Sigma-Aldrich and high purity water with a resistivity of 18.2 M$\Omega$.cm was obtained from an inline Millipore Synergy® UV water purification system. The water-propylene glycol mixtures at varying water content ranging from 0, 15, 30, 40, 50, 70 and 100 % were added to the vials to give a final solid concentration of 3.5 wt% and 0.5 wt% in the mixture and were stirred for at least 24 hours. The mixture was centrifuged and an aliquot is taken for PVA determination.

PVA content was measured via UV-Vis spectrophotometric method based on the above journal article using boric acid and iodine as follows: 500 µl of the aliquot was added to a 5-ml Eppendorf tube. 1500 µl of boric acid (0.04 g/ml) was added, and the solution was vortexed. 2000 µl distilled Millipore water was added to bring the final volume to 4000 µl. The solution was vortexed, and 1000 µl iodine solution was added, and vortexed. The solution was incubated for 15 minutes and the absorbance was measured at 590 nm. Samples were prepared in duplicate.

Results of the measurement are shown in Table 12. The overall solubility is expressed in two ways: in wt % of initial solid, meaning the percentage amount of the initial solid that was placed in the solvent mixture that dissolved, and in mg/ml, meaning the amount of solid in mg that dissolved in every ml of solvent. As water content is increased in the mixtures up to 50 %, PVA solubility increased from 0 to 19 wt % or equivalent to 0 to 6.6 mg /ml for the 3.5 wt % solids concentration level in mixture and from 0 to 21 wt % or equivalent to 0 to 1.0 mg/ml for the 0.5 wt % solids concentration level in mixture. These are interesting results as they indicate that though the fiber mat disintegrated, shrunk and/or disappeared as seen from the naked eye, not all are theoretically or quantitatively dissolved. Likely, the undissolved PVA that disappeared are too small to be seen by the naked eye.

The solubility behavior in water of the PVA fiber mats in comparison to PVA powders as obtained from the manufacturer was determined by visual inspection. ELVANOL 70-03 and ELVANOL 80-18 both as obtained from the manufacturer do not dissolve in water at room and cold temperature.

To dissolve ELVANOL 70-03 and ELVANOL 80-18, the water solvent as recommended is to be heated to at least 90 °C before PVA is added, and then the PVA-water mixture is recommended to be continuously heated with agitation. When transformed into fibers, it was surprisingly discovered that there was rapid fragmentation and eventual disappearance of the ELVANOL 70-03 and ELVANOL 80-18 fibers in water.

The solubility behavior of the fibers was determined by placing in a 150 ml beaker approximately 1.2 in x 1.2 in of square fiber section in high purity water with a resistivity of 18.2 MΩ-cm obtained from an in-line Millipore Synergy® UV water purification system at a PVA concentration of 0.1 wt%. The water was cooled in a recirculating bath (Thermo Electron Corporation, Neslar Merlin M25) filled with 50:50 water:ethylene glycol in a 250 mL-jacketed reaction kettle to the desired temperature and monitored with a 80PK-1 temperature probe connected to a digital readout display (Fluke 52II). The water cooled at the desired temperature was decanted and used in the visual solubility measurements. The temperature of the water-PVA mixture was monitored using an alcohol thermometer accurate to ±1 °C.

The fiber mat placed in water disappeared visually within 2 minutes at room temperature (~25°C), between 2.5-5 min at 15°C, between 3-7 min at 10°C, between 5-10 min at 5°C and between 10-20 min at close to 0°C.

Table 12 shows quantitative solubility measurements of ELVANOL 80-18 fiber mats and powder as received from Kuraray Co., Ltd using spectrophotometric method.

Table 12

| Sample | Propylene Glycol-Water Mixture Composition (%) | Overall Solubility (wt% based on wt of initial solid) | Overall Solubility (mg/ml) |
|---|---|---|---|
| **ELVANOL 80-18 fiber mat in 3.5 wt% solids in mixture (35 mg/ml)** | | | |
| *1* | 0% Water | 0.0% | 0.0 |
| *2* | 15% Water | 0.1% | 0.0 |
| *3* | 30% Water | 11.6% | 4.1 |
| *4* | 40% Water | 15.0% | 5.2 |
| *5* | 50% Water | 18.8% | 6.6 |
| *6* | 70% Water | 18.6% | 6.5 |
| *7* | 100% Water | 19.7% | 6.9 |
| **ELVANOL 80-18 fiber mat in 0.5 wt% solids in mixture (5 mg/ml)** | | | |
| *1* | 0% Water | 0.1% | 0.0 |
| *2* | 15% Water | 0.1% | 0.0 |
| *3* | 30% Water | 13.3% | 0.7 |
| *4* | 40% Water | 19.3% | 1.0 |
| *5* | 50% Water | 21.2% | 1.1 |
| *6* | 70% Water | 21.2% | 1.1 |

(continued)

| ELVANOL 80-18 fiber mat in 0.5 wt% solids in mixture (5 mg/ml) | | | |
|---|---|---|---|
| 7 | 100% Water | 19.3% | 1.0 |
| ELVANOL 80-18 Powder (as received) in 3.5 wt% solids in mixture (35 mg/ml) | | | |
| 1 | 0% Water | 0.0% | 0.0 |
| 2 | 15% Water | 0.1% | 0.0 |
| 3 | 30% Water | 0.1% | 0.0 |
| 4 | 40% Water | 0.2% | 0.1 |
| 5 | 50% Water | 0.2% | 0.1 |
| 6 | 70% Water | 0.4% | 0.1 |
| 7 | 100% Water | 0.8% | 0.3 |

Effect of Fiber Crystallinity and Size on Solubility

The encapsulated enzyme leakage can be controlled by controlling the PVA polymer solubility through such parameters as the PVA polymer crystallinity and fiber size.

The crystallinity of the fiber samples was measured using dynamic scanning calorimetry (DSC). To optimize the temperature and time of the drying process in the DSC cell, few experiments were performed on the samples at 75°C and 100°C with timing of 5 and 10 minutes. The optimal temperature and time for drying that was selected were 100°C and 10 minutes.

To measure the enthalpy of fusion of the dried PVA powder and fiber samples, DSC experiments were performed from 20 degrees C to 100 degrees C at 5°C/min. Samples were then held for 10 minutes at 100 degrees C before being cooled to 0 degrees C at 10°C/min. Experiments were then continued on the dried samples in one heat temperature profile from 0 degrees C to 250 degrees C at 10°C/min in $N_2$ atmosphere using a Q1000 DSC from TA Instruments.

Table 13 demonstrates the varying solubility of PVA fiber mats as a function of crystallinity at constant fiber diameter. Solubility decreases as fiber crytallinity increases. To ensure constant fiber diameter when measuring crystallinity, samples from the same fiber mat were annealed at 120 degrees C and 150 degrees C for an hour and crystallinity measurements of the annealed fiber mats were compared a reference unannealed fiber mat sample.

Table 13

| Sample | Glass Transition Temperature | Average Fiber Size | Initial Crystallinity | $H_2O$ Solubility | $H_2O$ Solubility |
|---|---|---|---|---|---|
| | Tg (°C) | (μm) | (%) | (wt % of initial **solid**) | (mg/ml) |
| ELVANOL 70-03 fibers | 80.4 | 0.278 ± 0.199 | 34.7% | 22.5 | 7.9 |
| ELVANOL 70-03 fibers-annealed @120°C | 81.0 | 0.278 ± 0.199 | 48.8% | 9.8 | 3.4 |
| ELVANOL 70-03 fibers-annealed @150° | 81.4 | 0.278 ± 0.199 | 54.2 | 1.5 | 0.5 |

Table 14 demonstrates the effect of solubility of PVA fibers as a function of fiber size at constant crystallinity. Solubility decreased as fiber sizes increased. The 20 micron sized fibers with similar crystallinity were prepared using wet spinning as described by Sakurada, I. Polyvinyl alcohol fibers. 1985, Marcel Dekker, Inc. NY.

Table 14

| Sample | Glass Transition Temperature | Initial Crystallinity | Average Fiber Size | H$_2$O Solubility | H$_2$O Solubility |
|---|---|---|---|---|---|
| | Tg (°C) | (%) | (μm) | (wt % of initial solid) | (mg/ml) |
| ELVANOL 80-18 fibers | 78.3 | 28.1% | 0.901 ± 0.8 | 18.8 | 6.6 |
| ELVANOL 80-18 fibers | 76.0 | 22.0% | 1.652 ± 0.7 | 16.3 | 5.7 |
| ELVANOL 80-18 wet-spun fibers | 79.9 | 31.6% | 20.0 ± 2.0 | 11.0 | 3.0 |

**Claims**

1. An enzyme delivery system comprising:

   a plurality of electroblown fibers comprising:

   a) a water soluble polymeric resin; and
   b) an enzyme;

   wherein the enzyme is encapsulated in the electroblown fibers and is present in an amount from 0.1 to 40 wt% based on total weight of the electroblown fibers;

   wherein percent of active enzyme after encapsulation is from 50 to 100%; and
   wherein at least a portion of the electroblown fibers have a fiber diameter of 25 microns or less.

2. The enzyme delivery system in accordance with claim 1, wherein the water soluble polymeric resin is methylcellulose, hydroxypropyl methylcellulose, guar gum, alginate, polyvinyl pyrrolidone, polyethylene oxide, polyvinyl alcohol, pullulan, polyaspartic acid, polyacrylic acid, copolymers thereof or mixtures thereof, and preferably is polyvinyl pyrrolidone, polyvinyl alcohol, pullulan or mixtures thereof.

3. The enzyme delivery system in accordance with claim 1, wherein the electroblown fibers have a crystallinity from 20 to 54%.

4. The enzyme delivery system in accordance with claim 1, wherein the electroblown fibers have a crystallinity less than 35%.

5. The enzyme delivery system in accordance with claim 1, wherein the enzyme is provided in a composition that is substantially free of cells or cell debris.

6. The enzyme delivery system in accordance with any of the preceding claims, wherein the enzyme is selected from: peroxidases, gluco-amylases, xylanases, hemicelluloses, cellulases, lipolytic enzymes, metallolipolytic enzymes, pectate lyases, metalloproteinase, amadoriase, arabinofuranosidases, phytases, isomerases, lipases, phospholipases, esterases, cutinases, pectinases, keratanases, reductases, oxidases, phenoloxidases, lipoxygenases, ligninases, pullulanases, tannases, pentosanases, malanases, beta-glucanases, arabinosidases, hyaluronidase, chondroitinase, dextranase, transferase, laccase, mannanase, xyloglucanases, or mixtures thereof.

7. The enzyme delivery system in accordance with any of the preceding claims, wherein the enzyme is selected from perhydrolase, protease, amylase or mixtures thereof.

8. The enzyme delivery system in accordance with any of the preceding claims, wherein the electroblown fibers form a woven web, fragmented woven web, a non-woven web, a fragmented non-woven web or individual fibers.

9. The enzyme delivery system in accordance with any of the preceding claims, wherein enzyme leakage is 21% or less after 200 days when the enzyme delivery system is suspended in an aqueous composition comprising 35 wt% water or less based on the total weight of the aqueous composition and wherein the aqueous composition is at a temperature from 20 to 30 degrees C, and is preferably 10% or less and more preferably 4% or less; and/or
wherein enzyme leakage is 21% or less after 14 days when the enzyme delivery system is suspended in an aqueous composition comprising 35 wt% water or less based on the total weight of the aqueous composition and wherein the aqueous composition is at a temperature from 20 to 30 degrees C; and/or
wherein enzyme leakage is less than 32% after 30 minutes when the enzyme delivery system is suspended in an aqueous composition comprising 70 wt% water or less based on total weight of the aqueous composition and wherein the aqueous composition is at a temperature from 20 to 30 degrees C.

10. The enzyme delivery system in accordance with any of the preceding claims, wherein the electroblown fibers have a solubility of 7.7 mg/mL or less in water at a temperature from 30 to 0 degrees C.

11. The enzyme delivery system in accordance with any of the preceding claims, further comprising a substrate, mediator, or cofactor for the enzyme.

12. The enzyme delivery system in accordance with any of the preceding claims wherein at least 90 wt% of the encapsulated enzyme is released into an aqueous composition at a temperature ranging from 0 to 30 degrees C in a time that is less than 15 minutes after the aqueous composition is diluted with water to contain greater than 70 weight percent water.

13. An aqueous composition comprising the enzyme delivery system in accordance with any of the preceding claims, wherein the aqueous composition comprises 70 wt% or less of water based on total weight of the aqueous composition.

14. Use of the enzyme delivery system in accordance with any of claims 1 to 12, in a liquid detergent composition comprising 70 wt% or less of water based on total weight of the liquid detergent composition and wherein the enzyme is any enzyme suitable for use in detergent compositions.

15. Use of the enzyme delivery system in accordance with any of claims 1 to 12, in a laundry prespotting composition comprising 70 wt% or less of water based on total weight of the laundry prespotting composition.

**Patentansprüche**

1. System zur Enzymfreisetzung, umfassend:

   eine Vielzahl von elektrogeblasenen Fasern, umfassend:

   a) ein wasserlösliches Polymerharz; und
   b) ein Enzym;

   wobei das Enzym in den elektrogeblasenen Fasern verkapselt ist und bezogen auf das Gesamtgewicht der elektrogeblasenen Fasern in einer Menge von 0,1 bis 40 Gew.-%, vorliegt;
   wobei der prozentuale Anteil des aktiven Enzyms nach der Verkapselung 50 bis 100% beträgt; und
   wobei mindestens ein Teil der elektrogeblasenen Fasern einen Faserdurchmesser von 25 Mikrometer oder weniger aufweist.

2. System zur Enzymfreisetzung nach Anspruch 1, wobei das wasserlösliche Polymerharz Methylcellulose, Hydroxypropylmethylcellulose, Guargummi, Alginat, Polyvinylpyrrolidon, Polyethylenoxid, Polyvinylalkohol, Pullulan, Polyasparaginsäure, Polyacrylsäure, Copolymere davon oder Mischungen davon ist, und vorzugsweise Polyvinylpyrrolidon, Polyvinylalkohol, Pullulan oder Mischungen davon ist.

3. System zur Enzymfreisetzung nach Anspruch 1, wobei die elektrogeblasenen Fasern eine Kristallinität von 20 bis 54% aufweisen.

4. System zur Enzymfreisetzung nach Anspruch 1, wobei die elektrogeblasenen Fasern eine Kristallinität von weniger als 35% aufweisen.

5. System zur Enzymfreisetzung nach Anspruch 1, wobei das Enzym in einer Zusammensetzung bereitgestellt wird, die im Wesentlichen frei ist von Zellen oder Zelltrümmern.

6. System zur Enzymfreisetzung nach einem der vorhergehenden Ansprüche, wobei das Enzym ausgewählt ist aus: Peroxidasen, Glucoamylasen, Xylanasen, Hemicellulosen, Cellulasen, lipolytischen Enzymen, metallolipolytischen Enzymen, Pektatlyasen, Metalloproteinase, Amadoriase, Arabinofuranosidasen, Phytasen, Isomerasen, Lipasen, Phospholipasen, Esterasen, Cutinasen, Pektinasen, Keratanasen, Reduktasen, Oxidasen, Phenoloxidasen, Lipoxygenasen, Ligninasen, Pullulanasen, Tannasen, Pentosanasen, Malanasen, Beta-Glucanasen, Arabinosidasen, Hyaluronidase, Chondroitinase, Dextranase, Transferase, Laccase, Mannanase, Xyloglucanasen oder Mischungen davon.

7. System zur Enzymfreisetzung nach einem der vorhergehenden Ansprüche, wobei das Enzym ausgewählt ist aus Perhydrolase, Protease, Amylase oder Mischungen davon.

8. System zur Enzymfreisetzung nach einem der vorhergehenden Ansprüche, wobei die elektrogeblasenen Fasern eine gewebte Bahn, eine fragmentierte gewebte Bahn, eine Vliesstoffbahn, eine fragmentierte Vliesstoffbahn oder einzelne Fasern bilden.

9. System zur Enzymfreisetzung nach einem der vorhergehenden Ansprüche, wobei der Enzymverlust nach 200 Tagen 21% oder weniger beträgt, wenn das System zur Enzymfreisetzung in einer wässrigen Zusammensetzung suspendiert ist, umfassend bezogen auf das Gesamtgewicht der wässrigen Zusammensetzung 35 Gew.-% Wasser oder weniger und wobei sich die wässrige Zusammensetzung bei einer Temperatur von 20° bis 30 °C befindet und bevorzugt 10% oder weniger und mehr bevorzugt 4% oder weniger beträgt;
und/oder
wobei der Enzymverlust nach 14 Tagen 21% oder weniger beträgt, wenn das System zur Enzymfreisetzung in einer wässrigen Zusammensetzung suspendiert ist, umfassend bezogen auf das Gesamtgewicht der wässrigen Zusammensetzung 35 Gew.-% Wasser oder weniger und wobei sich die wässrige Zusammensetzung bei einer Temperatur von 20° bis 30 °C befindet;
und/oder
wobei der Enzymverlust nach 30 Minuten weniger als 32% beträgt, wenn das System zur Enzymfreisetzung in einer wässrigen Zusammensetzung suspendiert ist, umfassend bezogen auf das Gesamtgewicht der wässrigen Zusammensetzung 70 Gew.-% Wasser oder weniger und wobei sich die wässrige Zusammensetzung bei einer Temperatur von 20° bis 30 °C befindet.

10. System zur Enzymfreisetzung nach einem der vorhergehenden Ansprüche, wobei die elektrogeblasenen Fasern eine Löslichkeit in Wasser bei einer Temperatur von 30° bis Null Grad Celsius von 7,7 mg/ml oder weniger aufweisen.

11. System zur Enzymfreisetzung nach einem der vorhergehenden Ansprüche, ferner umfassend ein Substrat, eine Mediatorsubstanz oder einen Cofaktor für das Enzym.

12. System zur Enzymfreisetzung nach einem der vorhergehenden Ansprüche, wobei mindestens 90 Gew.-% des verkapselten Enzyms in eine wässrige Zusammensetzung bei einer Temperatur im Bereich von 0° bis 30 °C in einer Zeit freigesetzt wird, die weniger als 15 Minuten beträgt, nachdem die wässrige Zusammensetzung mit Wasser verdünnt worden ist, um mehr als 70 Gew.-% Wasser zu enthalten.

13. Wässrige Zusammensetzung, umfassend das System zur Enzymfreisetzung nach einem der vorhergehenden Ansprüche, wobei die wässrige Zusammensetzung bezogen auf das Gesamtgewicht der wässrigen Zusammensetzung 70 Gew.-% oder weniger Wasser umfasst.

14. Verwendung des Systems zur Enzymfreisetzung nach einem der Ansprüche 1 bis 12 in einer flüssigen Waschmittelzusammensetzung, umfassend bezogen auf das Gesamtgewicht der flüssigen Waschmittelzusammensetzung 70 Gew.-% oder weniger Wasser, und wobei das Enzym jedes beliebige Enzym ist, das zur Verwendung in Waschmittelzusammensetzungen geeignet ist.

15. Verwendung des Systems zur Enzymfreisetzung nach einem der Ansprüche 1 bis 12 in einer Wäschevorentfleckungszusammensetzung, die 70 Gew.-% oder weniger Wasser, bezogen auf das Gesamtgewicht der Wäschevorentfleckungszusammensetzung, umfasst.

**Revendications**

1. Système de livraison d'enzyme comprenant:

   une pluralité de fibres électrosoufflées comprenant:

      a) une résine polymère soluble dans l'eau; et
      b) une enzyme;

   dans lequel l'enzyme est encapsulée dans les fibres électrosoufflées et est présente dans une quantité de 0,1 à 40% en poids sur la base du poids total des fibres électrosoufflées;
   dans lequel le pourcentage d'enzyme active après l'encapsulation est de 50 à 100%; et
   dans lequel au moins une portion des fibres électrosoufflées a un diamètre de fibre de 25 microns ou moins.

2. Système de livraison d'enzyme selon la revendication 1, dans lequel la résine polymère soluble dans l'eau est une méthylcellulose, une hydroxypropylméthylcellulose, une gomme de guar, un alginate, une polyvinylpyrrolidone, un polyéthylène oxyde, un alcool polyvinylique, un pullulane, un acide polyaspartique, un acide polyacrylique, des copolymères de ceux-ci ou des mélanges de ceux-ci et de préférence une polyvinylpyrrolidone, un alcool polyvinylique, un pullulane ou des mélanges de ceux-ci.

3. Système de livraison d'enzyme selon la revendication 1, dans lequel les fibres électrosoufflées ont une cristallinité de 20 à 54%.

4. Système de livraison d'enzyme selon la revendication 1, dans lequel les fibres électrosoufflées ont une cristallinité inférieure à 35%.

5. Système de livraison d'enzyme selon la revendication 1, dans lequel l'enzyme est fournie dans une composition qui est substantiellement exempte de cellules ou de débris cellulaires.

6. Système de livraison d'enzyme selon l'une quelconque des revendications précédentes, dans lequel l'enzyme est choisie parmi: peroxydases, gluco-amylases, xylanases, hémicelluloses, cellulases, enzymes lipolytiques, enzymes métallolipolytiques, pectate lyases, métalloprotéinase, amadoriase, arabinofuranosidases, phytases, isomérases, lipases, phospholipases, estérases, cutinases, pectinases, kératanases, réductases, oxydases, phénoloxydases, lipoxygénases, ligninases, pullulanases, tannases, pentosanases, malanases, bêta-glucanases, arabinosidases, hyaluronidase, chondroïtinase, dextranase, transférase, laccase, mannanase, xyloglucanases ou des mélanges de celles-ci.

7. Système de livraison d'enzyme selon l'une quelconque des revendications précédentes, dans lequel l'enzyme est choisie parmi: perhydrolase, protéase, amylase ou des mélanges de celles-ci.

8. Système de livraison d'enzyme selon l'une quelconque des revendications précédentes, dans lequel les fibres électrosoufflées forment un tissu tissé, un tissu tissé fragmenté, un tissu non tissé, un tissu non tissé fragmenté ou des fibres individuelles.

9. Système de livraison d'enzyme selon l'une quelconque des revendications précédentes, dans lequel la fuite d'enzyme est de 21% ou moins après 200 jours lorsque le système de livraison d'enzyme est en suspension dans une composition aqueuse comprenant 35% en poids d'eau ou moins sur la base du poids total de la composition aqueuse et dans lequel la composition aqueuse est à une température de 20 à 30 degrés C et est de préférence de 10% ou moins et plus préférablement de 4% ou moins;
   et/ou
   dans lequel la fuite d'enzyme est de 21% ou moins après 14 jours lorsque le système de livraison d'enzyme est en suspension dans une composition aqueuse comprenant 35% en poids d'eau ou moins sur la base du poids total de la composition aqueuse et dans lequel la composition aqueuse est à une température de 20 à 30 degrés C;
   et/ou
   dans lequel la fuite d'enzyme est inférieure à 32% après 30 minutes lorsque le système de livraison d'enzyme est en suspension dans une composition aqueuse comprenant 70% en poids d'eau ou moins sur la base du poids total de la composition aqueuse et dans lequel la composition aqueuse est à une température de 20 à 30 degrés C.

10. Système de livraison d'enzyme selon l'une quelconque des revendications précédentes, dans lequel les fibres électrosoufflées ont une solubilité de 7,7 mg/ml ou moins dans l'eau à une température de 30 à 0 degrés C.

11. Système de livraison d'enzyme selon l'une quelconque des revendications précédentes, comprenant en outre un substrat, un médiateur ou un cofacteur pour l'enzyme.

12. Système de livraison d'enzyme selon l'une quelconque des revendications précédentes, dans lequel au moins 90% en poids de l'enzyme encapsulée sont libérés dans une composition aqueuse à une température variant de 0 à 30 degrés C en un temps qui est inférieur à 15 minutes après la dilution de la composition aqueuse avec l'eau pour contenir plus de 70 pour cent en poids d'eau.

13. Composition aqueuse comprenant le système de livraison d'enzyme selon l'une quelconque des revendications précédentes, où la composition aqueuse comprend 70% en poids ou moins d'eau sur la base du poids total de la composition aqueuse.

14. Utilisation du système de livraison d'enzyme selon l'une quelconque des revendications 1 à 12, dans une composition de détergent liquide comprenant 70% en poids ou moins d'eau sur la base du poids total de la composition de détergent liquide et où l'enzyme est n'importe quelle enzyme appropriée pour une utilisation dans des compositions de détergent.

15. Utilisation du système de livraison d'enzyme selon l'une quelconque des revendications 1 à 12, dans une composition de prédétachant de blanchisserie comprenant 70% en poids ou moins d'eau sur la base du poids total de la composition de prédétachant de blanchisserie.

FIG. 1

100% PG     85/15     70/30     60/40     50/50     30/70     100% $H_2O$

Increasing water content

*FIG. 2*

Increasing water content

100% PG | 85/15 | 70/30 | 60/40 | 50/50 | 30/70 | 100% H$_2$O

*FIG. 3*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62262619
- WO 2015177077 A
- US 7618579 B
- US 7582247 B

**Non-patent literature cited in the description**

- **BRISCOE B ; LUCKHAM P ; ZHU S.** The effects of hydrogen bonding upon the viscosity of aqueous poly(vinyl alcohol) solutions. *Polymer,* 2000, vol. 41, 3851-3860
- **L. PROCHÁZKOVÁ ; Y. RODRÍGUEZ-MUÑOZ ; J. PROCHÁZKA ; J. WANNERA.** *Intern. J. Environ. Anal. Chem.,* 2014, vol. 94, 399-410
- **I.F. ALEKSANDROVICH ; L.N. LYUBIMOVA.** *Fibre Chem.,* 1993, vol. 24 (156
- **D.P. JOSHI ; Y.L. LAN-CHUN-FUNG ; J.G. PRITCHARD.** *Anal. Chim. Acta.,* 1979, vol. 104 (153
- **Y. MORISHIMA ; K. FUJISAWA ; S. NOZAKURA.** *Polym. J.,* 1978, vol. 10 (281
- **J.G. PRITCHARD ; D.A. AKINTOLA.** *Talanta,* 1972, vol. 19 (877
- **LAEMMLI, U. K.** Cleavage of structural proteins during the assembly of the head of bacteriophage T4. *Nature,* 1970, vol. 227 (5259), 680-685
- **PINKERNELL, U. ; EFFKEMANN, S. ; KARST, U.** Simultaneous HPLC determination of Peroxyacetic acid and hydrogen peroxide. *Anal. Chem.,* 1997, vol. 69 (17), 3623-3627
- **SAKURADA, I.** Polyvinyl alcohol fibers. Marcel Dekker, Inc, 1985